# EUROPEAN PATENT APPLICATION

(11) **EP 4 835 051 A1**
(43) Date of publication of application: **04.11.2026**
(21) Application number: 24912300.1
(22) Date of filing: 08.12.2024
(51) Int. Cl.: C12N 1/21, C12N 15/31, C12N 15/53, C12N 15/60, C12P 7/22, C12P 7/40

(54) **HYDROGENOPHILUS BACTERIUM TRANSFORMANT CAPABLE OF PRODUCING CIS, CIS-MUCONIC ACID OR CATECHOL**

(30) Priority: 28.12.2023 JP 2023223686
(71) Applicant: Utilization of Carbon Dioxide Institute Co.,Ltd., Tokyo 135-0091 (JP)
(72) Inventor: YUKAWA, Hideaki, Tokyo 135-0091 (JP)
(74) Representative: Müller-Boré & Partner Patentanwälte PartG mbB
(86) International application number: PCT/JP2024/043328
(87) International publication number: WO 2025/142411

(57) **Abstract**

*A Hydrogenophilus* bacterium transformant, in which a catechol 2,3-dioxygenase gene on its genome is disrupted, comprising (i) a 3-dehydroshikimate dehydratase gene and a protocatechuate decarboxylase gene that are exogenous, or (ii) a 3-dehydroshikimate dehydratase gene, a protocatechuate decarboxylase gene, and a catechol 1,2-dioxygenase gene that are exogenous is capable of producing muconic acid or catechol efficiently.

## Description

### Technical Field

The present invention relates to a *Hydrogenophilus* bacterium transformant having an ability to produce cis,cis-muconic acid or catechol, a method of producing cis,cis-muconic acid or catechol including using the transformant, and a *Hydrogenophilus* bacterium gene-disrupted strain suitable as a host for producing the transformant.

### Background Art

The Paris Agreement, which was adopted in 2015, provides that global emissions of greenhouse gases should be promptly reduced. Under the Agreement, Japan set the goal of reducing her emission of greenhouse gases such as carbon dioxide and methane by 46% compared with year 2013 levels, by the year 2030.

Globally, most chemical production depends on petroleum resources and thus contributes to increased greenhouse gas emissions. Accordingly, reducing dependence on petroleum is a desirable strategy for chemical production, and research and development of biorefineries, which produce green chemicals from biomass, are being actively conducted in various countries. However, biomass saccharification to produce raw materials for microbial fermentation requires complex processes and is also costly. In addition, the use of biomass that can serve as food or feed for chemical production disrupts the stable supply of food and feed. Furthermore, the large-scale use of biomass for chemical production may lead to environmental degradation.

As part of research on decarbonization, gases such as carbon dioxide, methane, and carbon monoxide have attracted attention as more sustainable carbon sources, and techniques for producing valuable substances and biofuels using microorganisms that utilize these gases are the subject of interest. In particular, carbon fixation and efficient utilization of carbon dioxide, a significant contributor to global warming, is highly anticipated.

Herein, cis,cis-muconic acid (hereinafter sometimes abbreviated as "muconic acid") has the following structure. 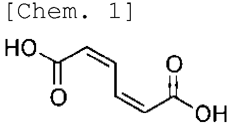 Muconic acid is an extremely useful dicarboxylic acid to be used as a starting material for producing various polymers, drugs, and the like because muconic acid has two double bonds and two carboxy groups in a molecule thereof, and hence has high reactivity. A muconic acid derivative using muconic acid as a starting material is, for example, adipic acid, terephthalic acid, or polyurethane. Adipic acid is a raw material for nylon-6,6, and terephthalic acid is a raw material for polyethylene terephthalate or polyethylene, and hence the acids have been used in large amounts in the production of daily necessities. Accordingly, such muconic acid derivatives are industrially important compounds.

A chemical process including oxidizing catechol with a copper catalyst or biological conversion using benzoic acid as a raw material has been known as a method of producing muconic acid. Catechol and benzoic acid serving as raw materials are derived from petroleum. In addition, most of the muconic acid derivatives have recently been produced by using petroleum resources. Specifically, adipic acid has been produced by oxidizing benzene-derived cyclohexane, and terephthalic acid has been produced by oxidizing xylene in petroleum in the presence of a cobalt-manganese-bromine-containing catalyst at 250°C. Those petroleum resource-dependent chemical synthesis processes each have an extremely large environmental load, and hence bio-based muconic acid production has been required as a substitute for the conventional chemical processes.

Catechol is a compound having the following structure. 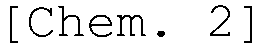 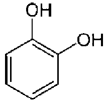 Catechol is used in the production of muconic acid. In addition, catechol has been used as an intermediate raw material for a perfume, a polymerization inhibitor, an antioxidant, a pharmaceutical, an agricultural chemical, or the like. Catechol has also been used as a raw material for: a peeling agent for a semiconductor photoresist to be used at the time of the production of a printed circuit board; an activated carbon adsorbent for deoxygenation; a plating treatment agent; a rubber vulcanizing agent; or the like.

Catechol has been mainly produced by oxidizing phenol with hydrogen peroxide water. Phenol serving as a raw material has been industrially produced by the cumene method using petroleum-derived benzene and petroleum-derived propylene as raw materials. The method is a complicated process. In addition, the method is a chemical industry process to be performed at high temperature and high pressure, and hence uses a large amount of energy. Accordingly, escape from a petroleum resource-dependent chemical synthesis process has been required.

In order to transition from a petroleum-dependent society, which faces concerns over future depletion of petroleum, toward a sustainable society, the production of chemical products by microbial fermentation using renewable, non-edible biomass resources has been attracting attention. However, as mentioned above, the use of biomass involves various challenges, including the need for complex processes, interference with the stable supply of food and feed, and significant environmental burden.

Accordingly, there is a demand for a practical method of producing muconic acid and catechol using microorganisms that does not interfere with the stable supply of food and feed, not imposing environmental burden, and enables production through simpler processes. In particular, there is a need for a practical method that can produce muconic acid and catechol by fixing carbon dioxide.

Protocatechuic acid, catechol, and muconic acid are present on the metabolic pathways of many microorganisms. In many microorganisms, protocatechuic acid is produced from 3-dehydroshikimic acid by the action of 3-dehydroshikimate dehydratase, and is also produced from 4-hydroxybenzoic acid by the action of 4-hydroxybenzoate hydroxylase. In addition, in many microorganisms, catechol is produced from protocatechuic acid by the action of protocatechuate decarboxylase, and is also produced from salicylic acid by the action of salicylate 1-monooxygenase. After that, the decomposition of catechol is advanced by its ortho cleavage or meta cleavage, and the decomposed product is incorporated into a TCA cycle. The ortho cleavage is advanced by catechol 1,2-dioxygenase to produce muconic acid. The meta cleavage is advanced by catechol 2,3-dioxygenase to produce 2-hydroxymuconate semialdehyde.

Patent Literature 1 discloses a technology of producing catechol from glucose through use of a transformed bacterium obtained by introducing a transketolase gene, a DAHP synthase gene, a 3-dehydroquinate synthase gene, *Klebsiella pneumoniae*-derived 3-dehydroshikimate dehydratase gene (*qsuB*), or *Klebsiella pneumoniae-*derived protocatechuate decarboxylase gene (*aroY*) into an *Escherichia* or *Klebsiella* bacterium serving as a host.

Patent Literature 2 discloses a technology of producing catechol from glucose through use of a transformed bacterium obtained by introducing *Lacticaseibacillus rhamnosus aroY* into a coryneform microorganism serving as a host, in which the functions of a protocatechuate dehydrogenase gene and a catechol 1,2-dioxygenase gene (*catA*) responsible for the ortho cleavage of catechol are reduced or deleted.

Patent Literature 3 discloses a technology of producing muconic acid by cleaving catechol, which has been synthesized via salicylic acid by using *Escherichia coli* suppressed in expression of a gene encoding a phosphotransferase-based enzyme, a gene encoding pyruvate kinase, and a gene encoding an enzyme that synthesizes L-phenylalanine, with *Pseudomonas putida* DOT-T1E-derived *catA*.

Patent Literature 4 discloses a technology of producing muconic acid by incorporating *Bacillus thuringiensis*-derived *qsuB*, *Klebsiella pneumoniae*-derived *aroY*, and *Pseudomonas putida*-derived catechol 1,2-dioxygenase gene (*catA*) into the chromosome of host *Escherichia coli* whose sugar metabolic pathway has been modified.

Patent Literature 5 discloses a technology of producing muconic acid from a lignin-derived aromatic compound or lignin-containing biomass by introducing *Klebsiella pneumoniae*-derived *aroY* into host *Pseudomonas putida*, in which a protocatechuate 3,4-dioxygenase gene and a muconate cycloisomerase gene that metabolizes muconic acid have been disrupted.

Patent Literature 6 discloses a technology of producing muconic acid through use of a transformant obtained by introducing *Klebsiella pneumoniae*-derived *qsuB* and *Klebsiella pneumoniae*-derived *aroY*, and *Acinetobacter baylyi* ADP1-derived *catA* into *Escherichia coli* whose sugar metabolic pathway and shikimate pathway have been modified.

Non Patent Literature 1 teaches a technology of producing catechol from glucose through use of a transformed bacterium obtained by introducing *Klebsiella pneumoniae aroY* into *Escherichia coli*.

Patent Literature 2 teaches a technology of producing muconic acid from glucose through use of a transformant obtained by introducing *Podospora anserina*-derived *qsuB*, *Klebsiella pneumoniae-*derived *aroY*, and *Candida albicans*-derived *catA* into *Saccharomyces cerevisiae*.

Non Patent Literature 3 teaches a technology of producing muconic acid from glucose through use of a transformant obtained by introducing *Podospora anserina*-derived *qsuB*, *Klebsiella pneumoniae-*derived *aroY*, and *Acinetobacter calcoaceticus*-derived *catA* into *Saccharomyces cerevisiae*.

Non Patent Literature 4 teaches a technology of producing muconic acid from glucose through use of a transformant obtained by introducing *Bacillus cereus*-derived *qsuB*, *Enterobacter cloacae-*derived *aroY*, and *Pseudomonas putida*-derived *catA* into *Pseudomonas putida* KT2440 in which a protocatechuate 3,4-dioxygenase gene and a muconate cycloisomerase gene have been disrupted.

Non Patent Literature 5 teaches a technology of producing muconic acid from glucose by causing *Corynebacterium glutamicum* 13032, in which a protocatechuate 3,4-dioxygenase gene and a muconate cycloisomerase gene have been disrupted, to express an endogenous *qsuB* and an exogenous protocatechuate decarboxylase gene different from *aroY*.

Non Patent Literature 6 teaches a technology of producing muconic acid from glucose by causing *Corynebacterium glutamicum* 13032, in which a protocatechuate 3,4-dioxygenase gene and a muconate cycloisomerase gene have been disrupted, to express endogenous *qsuB* and endogenous *catA*, and *Klebsiella pneumoniae aroY*.

Non Patent Literature 7 teaches a technology of producing muconic acid from glucose through use of a transformant obtained by introducing *Klebsiella pneumoniae*-derived *qsuB* and *Klebsiella pneumoniae*-derived *aroY*, and *Acinetobacter* ADP1-derived *catA* improved in enzymatic properties into *Escherichia coli*.

Non Patent Literature 8 teaches a technology of producing muconic acid from glucose by causing *Klebsiella pneumoniae*, whose sugar metabolic pathway has been modified, and in which a protocatechuate 3,4-dioxygenase gene and a muconate cycloisomerase gene have been disrupted, to express endogenous *catA*.

However, those methods are methods of producing muconic acid or catechol by growing a microorganism through utilization of a sugar, which is a carbon raw material to be produced at high cost, and are not methods of producing muconic acid or catechol by using carbon dioxide as a carbon raw material.

*Clostridium* bacteria that are anaerobic microorganisms grow by using a chlorine-containing gaseous substrate as a carbon source (carbon monoxide substantially serves as a carbon source), and the bacteria have been utilized in the production of chemical products. However, the utilization of the bacteria as hosts for the production of muconic acid and catechol has not been reported.

Among bacteria capable of using carbon dioxide as a carbon source for growth, *Cupriavidus necator* H16 strain, *Hydrogenobacter thermophilus* TK-6 strain, and the like have been used in the production of chemical products, but the use of these strains as hosts for muconic acid and catechol production has not been reported.

### Citation List

### Patent Literature

[Patent Literature 1] US 5272073 B
[Patent Literature 2] WO 2019/211937 A
[Patent Literature 3] WO 2017/033965 A
[Patent Literature 4] JP 2020-184993 A
[Patent Literature 5] WO 2020/080467 A
[Patent Literature 6] 2019-195330 A

### Non-Patent Literature

[Non Patent Literature 1] J. Am. Chem. Soc., 117(9):2395-2400 (1995)
[Non Patent Literature 2] Biotechnol Bioeng., 119(2):376-387 (2022)
[Non Patent Literature 3] FEMS Yeast Res., 18(2) (2018)
[Non Patent Literature 4] Nat Commun., 13(1):4925 (2022)
[Non Patent Literature 5] Biochem Biophys Res Commun., 499(2):279-284. (2018)
[Non Patent Literature 6] Sci Rep., 8(1):18041 (2018)
[Non Patent Literature 7] Sci Rep., 5:13435 (2015)
[Non Patent Literature 8] Appl Microbiol Biotechnol., 99(12):5217-5225 (2015)

### Summary of Invention

### Technical Problem

The objective of the present invention is to provide a *Hydrogenophilus* bacterium transformant, which can efficiently produce muconic acid or catechol through utilization of carbon dioxide as a sole carbon source, a method of efficiently producing muconic acid or catechol by using the transformant, and a *Hydrogenophilus* bacterium gene-disrupted strain suitable as a host for producing the transformant.

### Solution to Problem

The inventor of the present invention has focused on *Hydrogenophilus* bacteria as bacteria capable of fixing carbon dioxide on an industrial scale for the purpose of avoiding the use of high cost raw materials such as sugars and contributing to global warming countermeasures. H*ydrogenophilus* bacteria are bacteria which grow by producing organic substances from carbon dioxide by utilizing hydrogen energy. The growth rate of such bacteria is generally extremely slow, however, the growth rate of *Hydrogenophilus* bacteria is fast, and their ability of fixing carbon dioxide is remarkably higher than that of plants and photosynthetic bacteria.

*Hydrogenophilus* bacteria do not have genes each encoding an enzyme for producing muconic acid and catechol. Therefore, it is necessary to introduce the gene of an enzyme which catalyzes a reaction producing muconic acid and catechol to *Hydrogenophilus* bacteria in order to provide capability of producing muconic acid and catechol to *Hydrogenophilu*s bacteria.

However, even when an *aroY*, a *catA*, and a *qsuB* are introduced into a *Hydrogenophilus* bacterium and expressed, muconic acid or catechol has not been produced in an amount enough for industrial utilization.

The inventor of the present invention has found that although *Hydrogenophilus* bacteria are free of genes that are estimated to be a protocatechuate 3,4-dioxygenase gene and a muconate cycloisomerase gene unlike *Pseudomonas putida* described in Patent Literature 5, the bacteria have a gene that is estimated to encode catechol 2,3-dioxygenase that subjects catechol to meta cleavage to form 2-hydroxymuconate semialdehyde (corresponding to *HPTL_0060* in *Hydrogenophilus thermoluteolus*). In addition, the inventor of the present invention has conceived that when a catechol 2,3-dioxygenase gene functions, catechol produced in cells of *Hydrogenophilus* bacteria is metabolized to reduce the amount of muconic acid or catechol to be secreted into a medium supernatant.

Further, the inventor of the present invention has found that when a *qsuB* and an *aroY* are introduced into a strain in which the gene that is estimated to be the catechol 2,3-dioxygenase gene on the genome of *Hydrogenophilus* bacteria is disrupted, the amount of catechol to be secreted into a medium significantly increases. In addition, the inventor has found that when the *qsuB*, the *aroY*, and a catA are introduced into the strain in which the gene that is estimated to be catechol 2,3-dioxygenase gene on the genome of *Hydrogenophilus* bacteria is disrupted, the amounts of catechol and muconic acid to be secreted into the medium significantly increase. In view of the foregoing, the inventor has concluded that the disrupted gene is a catechol 2,3-dioxygenase gene (*xylE*), and in *Hydrogenophilus* bacteria, the disruption of the *xylE* is essential to efficient production of muconic acid or catechol.

In addition, the inventor of the present invention has found that an *xylE*-disrupted strain having introduced thereinto a *qsuB* and an *aroY* extremely efficiently produces catechol by using carbon dioxide as a sole carbon source, and that an *xylE*-disrupted strain having introduced thereinto the *qsuB*, the *aroY*, and a *catA* extremely efficiently produces catechol and muconic acid by using carbon dioxide as a sole carbon source.

The present invention has been completed on the basis of the above-mentioned findings and provides the following [1] to [9].
[1] A *Hydrogenophilus* bacterium gene-disrupted strain, comprising a disrupted catechol 2,3-dioxygenase gene.
[2] The *Hydrogenophilus* bacterium gene-disrupted strain according to [1], wherein the catechol 2,3-dioxygenase gene is any one of the following DNAs (a1) to (a5).
   (a1) A DNA comprising the nucleotide sequence of SEQ ID NO: 1
   (a2) A DNA comprising a nucleotide sequence that has 90% or more identity to SEQ ID NO: 1 and encoding a polypeptide having catechol 2,3-dioxygenase activity
   (a3) A DNA encoding a polypeptide comprising the amino acid sequence of SEQ ID NO: 2
   (a4) A DNA encoding a polypeptide comprising an amino acid sequence that has 90% or more identity to SEQ ID NO: 2, the polypeptide having catechol 2,3-dioxygenase activity
   (a5) A DNA encoding a polypeptide comprising an amino acid sequence that has 1 to 30 amino acids deleted, substituted, inserted, or added in the amino acid sequence of SEQ ID NO: 2, the polypeptide having catechol 2,3-dioxygenase activity
[3] A *Hydrogenophilus* bacterium transformant, in which a catechol 2,3-dioxygenase gene on its genome is disrupted, comprising (i) a 3-dehydroshikimate dehydratase gene and a protocatechuate decarboxylase gene that are exogenous, or (ii) a 3-dehydroshikimate dehydratase gene, a protocatechuate decarboxylase gene, and a catechol 1,2-dioxygenase gene that are exogenous.
[4] The *Hydrogenophilus* bacterium transformant according to [3], wherein the protocatechuate decarboxylase gene is any one of the following DNAs (b1) to (b5).
   (b1) A DNA comprising the nucleotide sequence of SEQ ID NO: 3
   (b2) A DNA comprising a nucleotide sequence that has 90% or more identity to SEQ ID NO: 3 and encoding a polypeptide having protocatechuate decarboxylase activity
   (b3) A DNA encoding a polypeptide comprising the amino acid sequence of SEQ ID NO: 4
   (b4) A DNA encoding a polypeptide comprising an amino acid sequence that has 90% or more identity to SEQ ID NO: 4, the polypeptide having protocatechuate decarboxylase activity
   (b5) A DNA encoding a polypeptide comprising an amino acid sequence that has 1 to 40 amino acids deleted, substituted, inserted, or added in the amino acid sequence of SEQ ID NO: 4, the polypeptide having protocatechuate decarboxylase activity
[5] The *Hydrogenophilus* bacterium transformant according to [3] or
[4], wherein the catechol 1,2-dioxygenase gene is any one of the following DNAs (c1) to (c5).
   (c1) A DNA comprising the nucleotide sequence of SEQ ID NO: 5
   (c2) A DNA comprising a nucleotide sequence that has 90% or more identity to SEQ ID NO: 5 and encoding a polypeptide having catechol 1,2-dioxygenase activity
   (c3) A DNA encoding a polypeptide comprising the amino acid sequence of SEQ ID NO: 6
   (c4) A DNA encoding a polypeptide comprising an amino acid sequence that has 90% or more identity to SEQ ID NO: 6, the polypeptide having catechol 1,2-dioxygenase activity
   (c5) A DNA encoding a polypeptide comprising an amino acid sequence that has 1 to 30 amino acids deleted, substituted, inserted, or added in the amino acid sequence of SEQ ID NO: 6, the polypeptide having catechol 1,2-dioxygenase activity
[6] The *Hydrogenophilus* bacterium transformant according to any one of [3] to [5], wherein the 3-dehydroshikimate dehydratase gene is any one of the following DNAs (d1) to (d5).
   (d1) A DNA comprising the nucleotide sequence of SEQ ID NO: 7
   (d2) A DNA comprising a nucleotide sequence that has 90% or more identity to SEQ ID NO: 7 and encoding a polypeptide having 3-dehydroshikimate dehydratase activity
   (d3) A DNA encoding a polypeptide comprising the amino acid sequence of SEQ ID NO: 8
   (d4) A DNA encoding a polypeptide comprising an amino acid sequence that has 90% or more identity to SEQ ID NO: 8, the polypeptide having 3-dehydroshikimate dehydratase activity
   (d5) A DNA encoding a polypeptide comprising an amino acid sequence that has 1 to 60 amino acids deleted, substituted, inserted, or added in the amino acid sequence of SEQ ID NO: 8, the polypeptide having 3-dehydroshikimate dehydratase activity
[7] The *Hydrogenophilus* bacterium transformant according to any one of [3] to [6], wherein the *Hydrogenophilus* bacterium is *Hydrogenophilus thermoluteolus*.
[8] A method of producing muconic acid or catechol, comprising a step of culturing the *Hydrogenophilus* bacterium transformant according to any one of [3] to [7].
[9] A positive selection marker for a *Hydrogenophilus* bacterium, comprising the following DNA (e1) or (e2).
   (e1) A DNA comprising the nucleotide sequence of SEQ ID NO: 11
   (e2) A DNA comprising a nucleotide sequence that has 90% or more identity to SEQ ID NO: 11 and encoding a polypeptide having activity of imparting bleomycin resistance to a *Hydrogenophilus* bacterium

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical products can be produced while global warming due to increased atmospheric carbon dioxide is suppressed.

Muconic acid and catechol are industrially important chemical products. However, processes for the chemical synthesis from petroleum resources are not desirable from the viewpoint of environmental protection. In contrast, the production of muconic acid and catechol with a microorganism that fixes carbon dioxide can simultaneously solve global warming due to increased atmospheric carbon dioxide, and the securement of muconic acid and catechol that are industrially required.

Bacteria that can grow by utilizing the chemical energy generated by the reaction of hydrogen with oxygen and by using carbon dioxide as the sole carbon source, can produce chemical products from a mixture of oxygen hydrogen, and carbon dioxide gases as a raw material. Therefore, the bacteria can achieve the efficient conversion of carbon dioxide into organic compounds and can be cultured in a simple culture medium. Typically, such bacteria grow slowly, but the growth rate of *Hydrogenophilus* bacteria serving as hydrogen-oxidizing bacteria is exceptionally high. The "Journal of Mitsubishi Research Institute No. 34 1999" describes *Hydrogenophilus* bacteria as follows: "Their proliferative capacity is so high that their carbon dioxide fixation ability cannot be compared with that of plants, which truly indicates the high carbon dioxide fixation ability of microorganisms."

The *Hydrogenophilus* bacterium gene-disrupted strain of the present invention in which an *xylE* on its genome is disrupted may be suitably used as a host for producing a transformant that is improved in muconic acid or catechol production ability by introducing a gene thereinto. A transformant obtained by introducing a *qsuB* and an *aroY* into the gene-disrupted strain of the present invention is significantly improved in catechol production ability, and a transformant obtained by introducing the *qsuB*, the *aroY*, and a *catA* into the gene-disrupted strain of the present invention is significantly improved in muconic acid production ability and catechol production ability. As described above, the *Hydrogenophilus* bacteria have a particularly excellent carbon dioxide fixation ability among organisms having a carbon dioxide fixation ability. Accordingly, the present invention has paved the way for the production of muconic acid and catechol on an industrial scale through utilization of carbon dioxide.

### Brief Description of Drawings

[Figure 1] Figure 1 is a diagram for describing processes for the construction of a marker cassette and the disruption of the catechol 2,3-dioxygenase gene of a host in Examples.
[Figure 2] Figure 2 is a diagram for describing the processes for the construction of the marker cassette and the disruption of the catechol 2,3-dioxygenase gene of the host in Examples.
[Figure 3] Figure 3 is a diagram for describing the processes for the construction of the marker cassette and the disruption of the catechol 2,3-dioxygenase gene of the host in Examples.

### Description of Embodiments

The present invention is described in detail below.

### (1) Catechol 2,3-dioxygenase gene-disrupted strain of Hydrogenophilus Bacterium

### Wild-type catechol 2,3-dioxygenase gene wild-type xylE)

A wild-type *Hydrogenophilus* bacterium has an *xylE* on its genome. The wild-type *Hydrogenophilus* bacterium has an ability to metabolize catechol into 2-hydroxymuconate semialdehyde by producing catechol 2,3-dioxygenase having activity.

A gene-disrupted strain of the present invention is a *Hydrogenophilus* bacterium in which the *xylE* on its genome is disrupted.

In the present invention, it does not need to be known that the *xylE* has been identified as an *xylE*, and the *xylE* only needs to be a DNA encoding a polypeptide having such catechol 2,3-dioxygenase activity as to subject catechol to meta cleavage to produce 2-hydroxymuconate semialdehyde.

The *xylE* of the wild-type *Hydrogenophilus* bacterium is, for example, any one of the following DNAs (a1) to (a5).
(a1) A DNA comprising the nucleotide sequence of SEQ ID NO: 1 (in particular, consisting of the nucleotide sequence of SEQ ID NO: 1)
(a2) A DNA comprising a nucleotide sequence that has 90% or more, 95% or more, 98% or more, or 99% or more identity to SEQ ID NO: 1 (in particular, consisting of the nucleotide sequence that has 90% or more, 95% or more, 98% or more, or 99% or more identity to SEQ ID NO: 1) and encoding a polypeptide having catechol 2,3-dioxygenase activity
(a3) A DNA encoding a polypeptide comprising the amino acid sequence of SEQ ID NO: 2 (in particular, consisting of the amino acid sequence of SEQ ID NO: 2)
(a4) A DNA encoding a polypeptide comprising an amino acid sequence that has 90% or more, 95% or more, 98% or more, or 99% or more identity to SEQ ID NO: 2 (in particular, consisting of the amino acid sequence that has 90% or more, 95% or more, 98% or more, or 99% or more identity to SEQ ID NO: 2), the polypeptide having catechol 2,3-dioxygenase activity
(a5) A DNA encoding a polypeptide comprising an amino acid sequence that has 1 to 30, 1 to 10, 1 to 5, or 1 to 3 amino acids, or 1 amino acid deleted, substituted, inserted, or added in the amino acid sequence of SEQ ID NO: 2 (in particular, consisting of the amino acid sequence that has 1 to 30, 1 to 10, 1 to 5, or 1 to 3 amino acids, or 1 amino acid deleted, substituted, inserted, or added in the amino acid sequence of SEQ ID NO: 2), the polypeptide having catechol 2,3-dioxygenase activity

SEQ ID NO: 1 is the nucleotide sequence of *HPTL_0060* gene of the wild strain of *Hydrogenophilus thermoluteolus*, and SEQ ID NO: 2 is the amino acid sequence of a polypeptide encoded by *HPTL_0060* gene of the wild strain of *Hydrogenophilus thermoluteolus*.

In the present invention, the fact that a test polypeptide has catechol 2,3-dioxygenase activity is recognized by: causing the test polypeptide to react with catechol; and detecting an increase in absorbance at 375 nm derived from 2-hydroxymuconate semialdehyde serving as a product.

### Disruption of catechol 2,3-dioxygenase gene (xylE)

In the present invention, the term "disruption of a catechol 2,3-dioxygenase gene" means that the activity of catechol 2,3-dioxygenase serving as a gene product of the gene is lower than that of a parental strain before the disruption of the gene. The activity of the gene product is preferably 50% or less, more preferably 10% or less, still more preferably 1% or less of the activity of the parental strain.

As the expression level of a gene reduces, the activity of a gene product reduces. The expression level of the gene may be measured by quantitative PCR method (qPCR) or next-generation sequencing method (NGS). In addition, even when part of the coding region or expression-regulating region of the gene is mutated, the gene may be normally expressed. However, even such case corresponds to the "disruption of the gene" as long as the activity of the gene product is lower than that of its parental strain.

The disruption of a gene can be performed by mutating (deletion, substitution, insertion, addition of nucleotide(s), or a combination thereof) the coding region of the gene or a gene expression-regulating region thereof such as a promoter region, the coding region or the gene expression-regulating region being present on the chromosome of a *Hydrogenophilus* bacterium. Gene-disrupting methods have been well known, and examples thereof include: a gene knockout method using homologous recombination; and a screening method based on random introduction of gene mutation through use of a mutagen and on the phenotype of the gene.

For example, the following may be performed: a disrupted *xylE* is produced by PCR or the like; the disrupted gene is introduced into a parental strain; and homologous recombination between the disrupted gene and a gene on a genome is caused to substitute an *xylE* on a chromosome with the disrupted gene.

A gene disruption method based on homologous recombination has been well known. The inventor of the present invention has found an *rpsL* gene (SEQ ID NO: 10) as a gene responsible for streptomycin sensitivity that functions in a *Hydrogenophilus* bacterium (in particular, in a streptomycin-resistant strain) and hence may be used as a counterselection marker. The use of the gene can disrupt the *xylE* of the *Hydrogenophilus* bacterium through homologous recombination.

A mutant of the present invention is preferably such that the coding region of an *xylE* is mutated. To mutate the coding region, typically, the entire region of each gene or a partial region thereof may be deleted. In addition, the gene may be disrupted by inserting some nucleotide, oligonucleotide, or polynucleotide into the inside of the gene. In addition, the entire or partial region of the gene may be substituted with another nucleotide, oligonucleotide, or polynucleotide. When the deletion, substitution, and/or insertion of a plurality of nucleotides is introduced, the mutation may be introduced at one site in the gene, or may be dispersedly introduced at a plurality of sites therein.

The number of nucleotides to be deleted, substituted, or inserted is preferably 3 or more. In addition, nucleotides whose number corresponds to 1% or more of the full length of a gene are preferably deleted, substituted, or inserted. Thus, each gene can be reliably disrupted. The deletion or the substitution may be performed over the full length of the gene (i.e., 100% of nucleotides for forming the gene may be deleted or substituted). The number of nucleotides to be inserted may be set to 100,000 or less.

Except when the entire region of the *xylE* is deleted, the above-mentioned mutation is desirably introduced at a site except a region encoding the vicinity of the C-terminus of catechol 2,3-dioxygenase. This facilitates the loss or lowering of the function of the *xylE*. For example, the above-mentioned mutation is desirably introduced into a region encoding 95% or less with respect to the entire region from the N-terminus of catechol 2,3-dioxygenase.

A method of disrupting the *xylE* is described below by taking, as an example, a method of deleting the *xylE*.

It is appropriate that the DNA of the 5' upstream region of a region to be deleted of a DNA fragment consisting of the *xylE* of a *Hydrogenophilus* bacterium, and the DNA of the 3' downstream region of the region to be deleted be each amplified by performing a PCR through use of the genomic DNA of the *Hydrogenophilus* bacterium as a template, and these regions be linked to each other. The DNA obtained by linking the 5' upstream region and 3' downstream region of the region to be deleted to each other is DNA in which the *xylE* has been completely deleted.

To improve homologous recombination efficiency, the DNA of the 5' upstream region and the DNA of the 3' downstream region described above are each preferably a DNA of 10 or more nucleotides. Alternatively, the DNAs to be linked to each other do not need to consist of nucleotide sequences completely identical to the 5' upstream region of the region to be deleted and the 3' downstream region of the region to be deleted, respectively, and as the lengths of the regions to be used for homologous recombination increase, homologous recombination can be caused with lower identity to the 5' upstream region or 3' downstream region of the region to be deleted.

Then, a marker cassette to be inserted between the 5' upstream region and 3' downstream region of the *xylE* is produced. Herein, the marker cassette is such that a gene responsible for streptomycin sensitivity that functions in the *Hydrogenophilus* bacterium and a bleomycin resistance gene (SEQ ID NO: 11) that functions in the *Hydrogenophilus* bacterium are linked to each other adjacently or with a DNA of about 10,000 nucleotide pairs or less being interposed therebetween.

Then, it is appropriate that the marker cassette be inserted between the DNA fragments of the 5' upstream region and 3' downstream region of the *xylE*.

When gene linkage is performed so that, for example, primers that amplify the 5' upstream region and the 3' downstream region may each have a sequence homologous to that of a marker cassette gene, there is obtained a labeling DNA fragment having inserted thereinto a marker cassette in which a DNA fragment containing the gene responsible for streptomycin sensitivity and a DNA fragment containing the bleomycin resistance gene are linked by a PCR method.

An operation of introducing the labeling DNA fragment into the streptomycin-resistant strain of a *Hydrogenophilus* bacterium is performed. When the above-mentioned labeling DNA fragment is introduced onto the chromosome of the *Hydrogenophilus* bacterium, the bacterium shows streptomycin sensitivity and bleomycin resistance. Accordingly, a recombinant in which the *xylE* of the streptomycin-resistant strain has been substituted with the above-mentioned labeling DNA fragment may be selected by using the fact that the bacterium has streptomycin sensitivity and bleomycin resistance as an indicator.

Next, to eliminate the marker cassette substituted instead of the *xylE*, homologous recombination is performed by using the DNA fragment produced in advance from which the *xylE* has been deleted.

An operation of introducing the linear DNA fragments which contains the 5' upstream region and the 3' downstream region and from which the *xylE* has been deleted into the above-mentioned streptomycin-sensitive strains (recombinants in each of which the *xylE* has been substituted with the labeling DNA fragment) is performed, and a strain that has become streptomycin-resistant may be selected. A strain that is streptomycin-resistant and has lost bleomycin resistance is preferably selected. Thus, a recombinant from which the marker cassette inserted into the *xylE* has been eliminated, that is, a recombinant from which the xylE has been deleted is obtained.

The deletion of the gene may be recognized by determining the nucleotide sequence of the *xylE* of the resultant recombinant or by identifying the amplification size of the *xylE* by a PCR method.

The following DNA (e1) or (e2) functions in a *Hydrogenophilus* bacterium, and hence may be used as a positive selection marker.
(e1) A DNA comprising the nucleotide sequence of SEQ ID NO: 11 (in particular, DNA consisting of the nucleotide sequence of SEQ ID NO: 11)
(e2) A DNA comprising a nucleotide sequence that has 90% or more, 95% or more, 98% or more, or 99% or more identity to SEQ ID NO: 11 and encoding a polypeptide having activity of imparting bleomycin resistance to a *Hydrogenophilus* bacterium

In the present invention, the fact that a test DNA has activity of imparting bleomycin resistance to a *Hydrogenophilus* bacterium is recognized by the fact that the growth of the *Hydrogenophilus* bacterium having introduced thereinto the test DNA in the presence of 50 µg/mL of bleomycin is improved as compared to that before the introduction, for example, the fact that the *Hydrogenophilus* bacterium can grow in the presence of 50 µg/mL of bleomycin unlike the *Hydrogenophilus* bacterium before the introduction of the test DNA.

### (2) Transformant of catechol 2,3-dioxygenase gene-disrupted strain

A transformant (transformed cell) of the present invention is a *Hydrogenophilus* bacterium transformant, in which *xylE* on its genome is disrupted, and which includes a *qsuB* and an *aroY* that are exogenous, or the *qsuB*, the *aroY*, and a *catA* that are exogenous.

The transformant of the present invention may be a transformant obtained by introducing the *qsuB* and the *aroY*, or the *qsuB*, the *aro*Y, and the *catA* after the disruption of the *xylE* on the genome of a *Hydrogenophilus* bacterium, or may be a transformant obtained by disrupting the *xylE* on the genome after the introduction of the *qsuB* and the *aroY*, or the *qsuB*, the *aroY*, and the *catA* into the *Hydrogenophilus* bacterium.

It does not need to be known that the *aroY*, the *catA*, and the *qsuB* to be used in the present invention have been identified as an *aroY*, a *catA*, and a *qsuB*, respectively, and the *aroY*, the *catA*, and the *qsuB* only need to be a DNA encoding a polypeptide having protocatechuate decarboxylase activity, a DNA encoding a polypeptide having catechol 1,2-dioxygenase activity, and a DNA encoding a polypeptide having 3-dehydroshikimate dehydratase activity, respectively.

The protocatechuate decarboxylase activity is enzymatic activity by which catechol is produced from protocatechuic acid, the catechol 1,2-dioxygenase activity is enzymatic activity by which muconic acid is produced from catechol, and the 3-dehydroshikimate dehydratase activity is enzymatic activity by which protocatechuic acid is produced from 3-dehydroshikimic acid.

In addition, in the present invention, the *aroY*, the *catA*, and the *qsuB* may be the DNAs of an *aroY*, a *catA*, and a *qsuB* isolated from naturally occurring bacteria, respectively, or may be DNAs artificially synthesized through use of a method known by a person skilled in the art.

### Protocatechuate decarboxylase gene (aroY)

In the present invention, any one of the following DNAs (b1) to (b5) is preferably used as an *aroY*.
(b1) A DNA comprising the nucleotide sequence of SEQ ID NO: 3 (in particular, consisting of the nucleotide sequence of SEQ ID NO: 3)
(b2) A DNA comprising a nucleotide sequence that has 90% or more, 95% or more, 98% or more, or 99% or more identity to SEQ ID NO: 3 (in particular, consisting of the nucleotide sequence that has 90% or more, 95% or more, 98% or more, or 99% or more identity to SEQ ID NO: 3) and encoding a polypeptide having protocatechuate decarboxylase activity
(b3) A DNA encoding a polypeptide comprising the amino acid sequence of SEQ ID NO: 4 (in particular, consisting of the amino acid sequence of SEQ ID NO: 4)
(b4) A DNA encoding a polypeptide comprising an amino acid sequence that has 90% or more, 95% or more, 98% or more, or 99% or more identity to SEQ ID NO: 4 (in particular, consisting of the amino acid sequence that has 90% or more, 95% or more, 98% or more, or 99% or more identity to SEQ ID NO: 4), the polypeptide having protocatechuate decarboxylase activity
(b5) A DNA encoding a polypeptide comprising an amino acid sequence that has 1 to 40, in particular, 1 to 30, 1 to 10, 1 to 5, or 1 to 3 amino acids, or 1 amino acid deleted, substituted, inserted, or added in the amino acid sequence of SEQ ID NO: 4 (in particular, consisting of the amino acid sequence that has 1 to 40, in particular, 1 to 30, 1 to 10, 1 to 5, or 1 to 3 amino acids, or 1 amino acid deleted, substituted, inserted, or added in the amino acid sequence of SEQ ID NO: 4), the polypeptide having protocatechuate decarboxylase activity

SEQ ID NO: 3 is the nucleotide sequence of the *aroY* of *Enterobacter asburiae*, and SEQ ID NO: 4 is the amino acid sequence of a polypeptide encoded by the *aroY* of *Enterobacter asburiae*.

In the present invention, the fact that a test polypeptide has protocatechuate decarboxylase activity is recognized by: causing the test polypeptide to react with protocatechuic acid; and detecting produced catechol through use of HPLC or the like.

### Catechol 1,2-dioxygenase gene (catA)

In the present invention, any one of the following DNAs (c1) to (c5) is preferably used as a *catA*.
(c1) A DNA comprising the nucleotide sequence of SEQ ID NO: 5 (in particular, consisting of the nucleotide sequence of SEQ ID NO: 5)
(c2) A DNA comprising a nucleotide sequence that has 90% or more, 95% or more, 98% or more, or 99% or more identity to SEQ ID NO: 5 (in particular, consisting of the nucleotide sequence that has 90% or more, 95% or more, 98% or more, or 99% or more identity to SEQ ID NO: 5) and encoding a polypeptide having catechol 1,2-dioxygenase activity
(c3) A DNA encoding a polypeptide comprising the amino acid sequence of SEQ ID NO: 6 (in particular, consisting of the amino acid sequence of SEQ ID NO: 6)
(c4) A DNA encoding a polypeptide comprising an amino acid sequence that has 90% or more, 95% or more, 98% or more, or 99% or more identity to SEQ ID NO: 6 (in particular, consisting of the amino acid sequence that has 90% or more, 95% or more, 98% or more, or 99% or more identity to SEQ ID NO: 6), the polypeptide having catechol 1,2-dioxygenase activity
(c5) A DNA encoding a polypeptide comprising an amino acid sequence that has 1 to 30, 1 to 10, 1 to 5, or 1 to 3 amino acids, or 1 amino acid deleted, substituted, inserted, or added in the amino acid sequence of SEQ ID NO: 6 (in particular, consisting of the amino acid sequence that has 1 to 30, 1 to 10, 1 to 5, or 1 to 3 amino acids, or 1 amino acid deleted, substituted, inserted, or added in the amino acid sequence of SEQ ID NO: 6), the polypeptide having catechol 1,2-dioxygenase activity

SEQ ID NO: 5 is the nucleotide sequence of *Pseudomonas thermotolerans catA*, and SEQ ID NO: 6 is the amino acid sequence of a polypeptide encoded by *Pseudomonas thermotolerans catA*.

In the present invention, the fact that a test polypeptide has catechol 1,2-dioxygenase activity is recognized by: causing the test polypeptide to react with catechol; and detecting produced muconic acid through use of HPLC or the like.

### 3-Dehydroshikimate dehydratase gene (qsuB)

In the present invention, any one of the following DNAs (d1) to (d5) is preferably used as a *qsuB*.
(d1) A DNA comprising the nucleotide sequence of SEQ ID NO: 7 (in particular, consisting of the nucleotide sequence of SEQ ID NO: 7)
(d2) A DNA comprising a nucleotide sequence that has 90% or more, 95% or more, 98% or more, or 99% or more identity to SEQ ID NO: 7 (in particular, consisting of the nucleotide sequence that has 90% or more, 95% or more, 98% or more, or 99% or more identity to SEQ ID NO: 7) and encoding a polypeptide having 3-dehydroshikimate dehydratase activity
(d3) A DNA encoding a polypeptide comprising the amino acid sequence of SEQ ID NO: 8 (in particular, consisting of the amino acid sequence of SEQ ID NO: 8)
(d4) A DNA encoding a polypeptide comprising an amino acid sequence that has 90% or more, 95% or more, 98% or more, or 99% or more identity to SEQ ID NO: 8 (in particular, consisting of the amino acid sequence that has 90% or more, 95% or more, 98% or more, or 99% or more identity to SEQ ID NO: 8), the polypeptide having 3-dehydroshikimate dehydratase activity
(d5) A DNA encoding a polypeptide comprising an amino acid sequence that has 1 to 60, 1 to 30, 1 to 10, 1 to 5, or 1 to 3 amino acids, or 1 amino acid deleted, substituted, inserted, or added in the amino acid sequence of SEQ ID NO: 8 (in particular, consisting of the amino acid sequence that has 1 to 60, 1 to 30, 1 to 10, 1 to 5, or 1 to 3 amino acids, or 1 amino acid deleted, substituted, inserted, or added in the amino acid sequence of SEQ ID NO: 8), the polypeptide having 3-dehydroshikimate dehydratase activity

SEQ ID NO: 7 is the nucleotide sequence of *Pseudomonas thermotolerans qsuB*, and SEQ ID NO: 8 is the amino acid sequence of a polypeptide encoded by *Pseudomonas thermotolerans qsuB*.

In the present invention, the fact that a test polypeptide has 3-dehydroshikimate dehydratase activity is recognized by: causing the test polypeptide to react with 3-dehydroshikimic acid; and detecting produced protocatechuic acid through use of HPLC or the like.

In the present invention, the identities of nucleotide sequences and amino acid sequences were calculated using GENETYX ver. 17 (GENETYX).

In the present invention, a DNA comprising a nucleotide sequence that has 90% or more but less than 100% identity to the nucleotide sequence of a given DNA and encoding a polypeptide having the same type of activity as the polypeptide encoded by the given DNA is called a "homolog" of the given DNA. A DNA encoding a polypeptide comprising an amino acid sequence that has 90% or more but less than 100% identity to the amino acid sequence of a given polypeptide and having the same type of activity as the given polypeptide is called a homolog of the DNA encoding the given polypeptide. A DNA encoding a polypeptide comprising an amino acid sequence that has 1 to 60 amino acids deleted, substituted, inserted, or added in the amino acid sequence of a given polypeptide and having the same type of activity as the given polypeptide is also called a homolog of the DNA encoding the given polypeptide.

### Hydrogenophilus bacteria

Examples of *Hydrogenophilus* bacteria include *Hydrogenophilus thermoluteolus*, *Hydrogenophilus halorhabdus*, *Hydrogenophilus denitrificans*, *Hydrogenophilus hirschii*, *Hydrogenophilus islandicus*, *Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977))(It proliferates double per hour.). H*ydrogenophilus thermoluteolus* strain NBRC 14978 is internationally deposited under the Budapest Treaty, and is thus publicly available.

The hosts *Hydrogenophilus* bacteria may be bacteria isolated from nature or genetically modified bacteria derived from bacteria isolated from nature. Genetic modification may be intended, for example, to allow high expression of a gene, or isochorismate synthase gene and isochorismate pyruvate lyase gene.

Such a genetic modification can be made, for example, by removing (curing) endogenous plasmids in *Hydrogenophilus* bacteria, or other techniques. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Method of preparing transformant

A method including introducing a *qsuB* and an *aroY,* or the *qsuB*, the *aroY*, and a *catA* into a *Hydrogenophilus* bacterium or the *xylE-*disrupted strain of the *Hydrogenophilus* bacterium serving as a host to provide a transformant is described.

The introduction of those genes into the host can be performed by using common methods for introducing exogenous genes into bacteria. Those genes may be directly introduced into the host. Alternatively, those genes may be incorporated into a vector for transformation (e.g., plasmid vector, viral vector, cosmid, fosmid, or BAC) and the resulting vector may be introduced into the host.

When those genes are introduced into the host, the genes may be introduced by being cloned into the same plasmid vector, or may be introduced by being cloned into different plasmid vectors.

In addition, those genes may each be incorporated onto the genome of the host by homologous recombination or the like.

### Method for producing transformant

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 (SEQ ID NO: 9) is preferable. Those skilled in the art can prepare pCAMO-6 according to "Examples". Those skilled in the art can prepare pCAMO-6 by utilizing a gene synthesis service or the like based on its DNA sequence.

Examples of promoters in vectors include *tac* promoter, lac promoter, *trc* promoter, or each of promoters *OXB1* and *OXB11* to *OXB2*0 from Oxford Genetics Ltd. Examples of terminators in vectors include the *T1T2* terminator of *Escherichia coli* rRNA operon *rrnB*, the *t0* transcription terminator of bacteriophage λ, *T7* terminator, and the like.

Introduction of a gene into a *Hydrogenophilus* bacterium (transformation) can be carried out by publicly known methods such as calcium chloride method, calcium phosphate method, rubidium chloride method, and electric pulse method (electroporation method).

### (2) Method for producing muconic acid or catechol

The present invention provides a method for producing muconic acid or catechol with use of the transformant of the present invention described above. The method includes a step of culturing the transformant of the present invention, especially, a step of culturing the transformant of the present invention using an inorganic or organic culture medium while supplying a gas containing carbon dioxide, preferably a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that muconic acid or catechol can be produced efficiently.

*Hydrogenophilus* bacteria can grow using hydrogen as a source of energy and using carbon dioxide as a sole carbon source, and thus, *Hydrogenophilus* bacteria can fix carbon dioxide efficiently when muconic acid or catechol is produced using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably muconic acid or catechol can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into an airtight culture container and shaking culture can be carried out, or the transformant can be cultured using an airtight culture container while introducing mixed gases into the culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and muconic acid or catechol can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and muconic acid or catechol can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and muconic acid or catechol can be produced efficiently.

When the culture is performed as described above, muconic acid or catechol is produced in a culture liquid. Although muconic acid or catechol may be recovered by recovering a reaction mixture, the recovery may be performed by a method typically used in the art, and a method therefor is not particularly limited. For example, methods such as filtration, centrifugation, salting-out method, recrystallization method, an organic solvent extraction method, esterification distillation separation method, chromatography separation method, or electrodialysis method, are available as required. Those methods may be used alone or in combination thereof.

### Examples

Next, the present invention is described with reference to Examples. The technical scope of the present invention is not limited by the following Examples.

### (1) Acquisition of streptomycin-resistant strain

*Hydrogenophilus thermoluteolus* TH-1 strain (NBRC 14978) (hereinafter sometimes referred to as "TH-1 strain") was inoculated with a platinum loop into 5 mL of liquid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O dissolved in 1 L of distilled water (pH 7.0)] in a test tube, and the test tube was filled with a gas mixture of H₂:O₂:CO₂ = 7.5:1:1.5, followed by shaking culture at 52°C. The culture liquid after 24 hours was applied to solid LB medium containing 100 µg/ml of streptomycin, followed by culture at 52°C for 72 hours.

As a result, the formation of three colonies was able to be recognized on the solid LB medium containing 100 µg/ml of streptomycin. Those colonies were inoculated with a platinum loop into 5 mL of the liquid A medium containing 100 µg/ml of streptomycin in a test tube, and the test tube was filled with a gas mixture of H₂:O₂:CO₂ = 7.5:1:1.5, followed by culture at 52°C. After 24 hours, the growth of bacteria was observed. Accordingly, those strains were the streptomycin-resistant strains of TH-1 strain, and one of the strains was named SR88 strain.

### (2) Methods for PCR, electrophoresis, DNA recovery, and seamless cloning in constructions of marker cassette and expression plasmids

PCR, electrophoresis, DNA recovery, and the preparation of plasmid vector pCAMO-6 for seamless cloning in the construction of marker cassettes and expression plasmids were performed using the methods described below.

### (2-1) PCR, electrophoresis, and DNA recovery

PCR was performed in the usual manner using DNA Thermal Cycler manufactured by Life Technologies Inc. and using KOD One PCR Master Mix (manufactured by Toyobo Co., Ltd.) as a reaction reagent. After that, the resulting reaction mixture was subjected to electrophoresis on a 1% agarose gel. Appropriate DNA fragments were recovered from the gel using GEL/PCR Purification Mini Kit (FAVORGEN) as needed.

### (2-2) Preparation of plasmid vector pCAMO-6 for seamless cloning

For seamless cloning, plasmid vector pCAMO-6 was amplified by PCR using the DNA of SEQ ID NO: 9 as a template. The primers shown below were used for PCR.

### Primers for amplification of plasmid vector pCAMO-6

| | | |
|---|---|---|
| (a-1) | 5'-GAATTCGAGCTCCGTCGACA-3' | (SEQ ID NO: 12) |
| (b-1) | 5'-ATGCGTTTCTCCTCCAGATC-3' | (SEQ ID NO: 13) |

The electrophoresis results showed that an approximately 5.2-kbp DNA fragment corresponding to the vector gene was detected. The DNA fragment was recovered from the gel.

### (2-3) Joining of vector pCAMO-6 and other DNA fragments

The DNA fragment containing vector pCAMO-6 synthesized above was joined to each insert DNA fragment in a one-to-one manner by the recombinase extracted from *Escherichia coli* strain JM109.

*Escherichia coli* strain JM109 was transformed separately with each resulting reaction mixture by heat shock method, applied to LB medium containing 50 µg/mL kanamycin, and cultured at 37°C for 24 hours.

### (2-4) Plasmid extraction and gene sequence confirmation

Strains grown on the LB medium were individually inoculated into 5 mL of liquid LB medium containing 50 µg/mL kanamycin per test tube using a platinum loop, and cultured with shaking at 37°C. Plasmid DNA was extracted from each culture liquid. The analysis of the sequences of the genes inserted in the plasmids was outsourced to Eurofins Genomics K.K. and performed by Sanger method. The results confirmed that the gene sequences were identical to the corresponding sequences in a database.

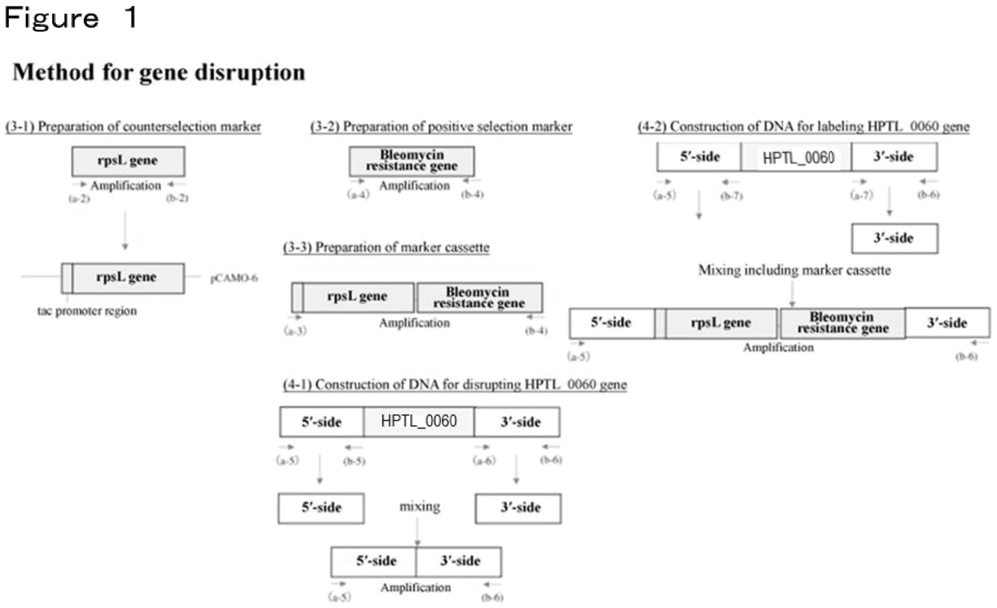

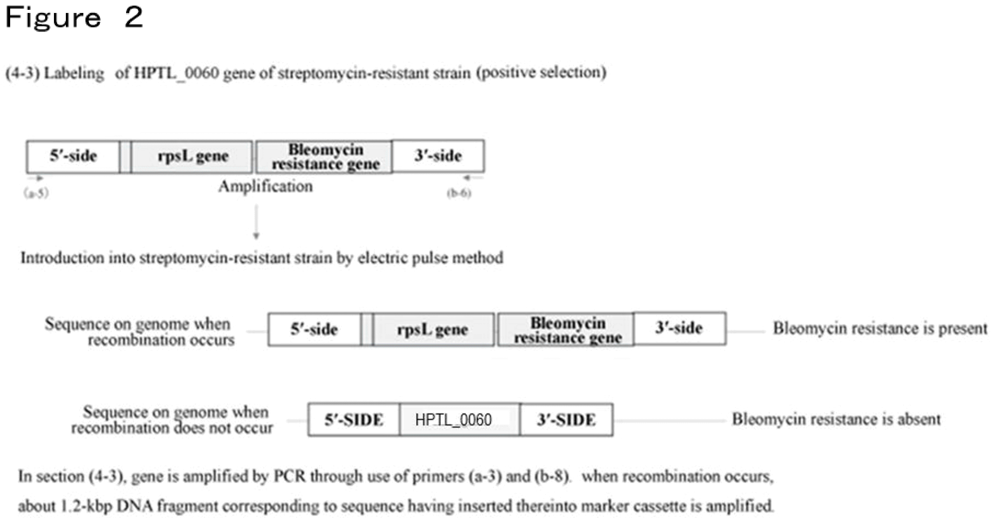

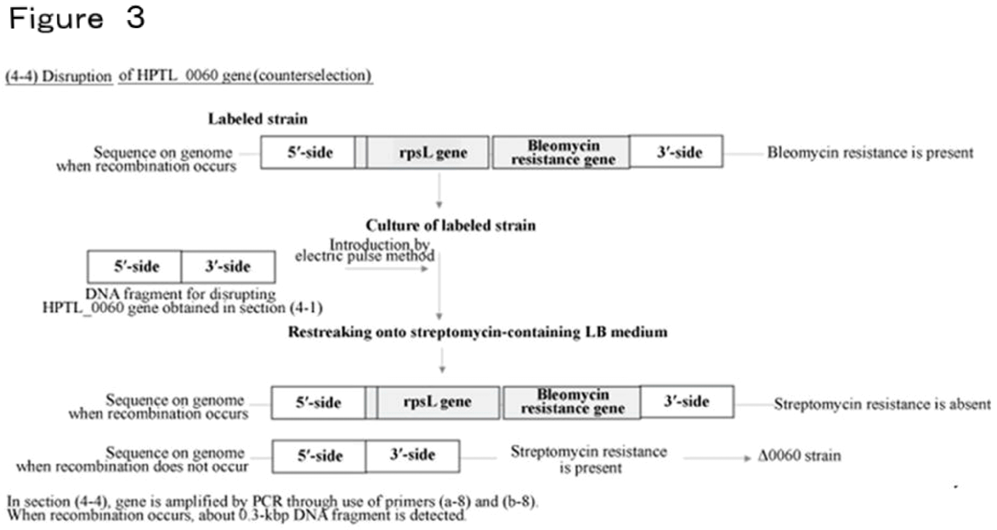

Processes for the construction of the marker cassette and the disruption of the catechol 2,3-dioxygenase gene of a host are illustrated in Figure 1 to Figure 3.

### (3) Construction of marker cassette

### (3-1) Preparation of counterselection marker

Genomic DNA was extracted from the wild strain (streptomycin-sensitive strain) of the TH-1 strain in accordance with a conventional method. Through use of the extracted genomic DNA as a template, a DNA fragment containing an *rpsL* gene, which was a gene responsible for streptomycin sensitivity, encoding ribosomal protein S12, was amplified by a PCR method. The following primers were used in the PCR.

### Primers for amplifying wild-type rpsL gene of TH-1 strain

| | | |
|---|---|---|
| (a-2) | 5'-CTGGAGGAGAAACGCATATGCCAACCATCAACCAGTTGGTG-3' | (SEQ ID NO: |
| | | 14) |
| (b-2) | 5'-CGACGGAGCTCGAATTCTTATTTCTTGCCCGCAGCGGC-3' | (SEQ ID NO: 15) |

A sequence homologous to that of vector pCAMO-6 is added to each of primer (a-2) and primer (b-2).

The resulting reaction mixture was subjected to gel electrophoresis. As a result, an about 0.4-kbp DNA fragment corresponding to the *rpsL* gene of the wild strain of TH-1 strain was detected, and the DNA fragment was recovered from the gel.

The DNA fragment of vector pCAMO-6 and the DNA fragment of the *rpsL* were ligated to each other to transform *Escherichia coli* JM109 strain. After that, plasmid extraction was performed to identify the sequence of the *rpsL* gene.

The *rpsL* gene including a *tac* promoter region was amplified by using pCAMO-6 having introduced thereinto the *rpsL* gene as a template.

### Primers for amplifying rpsL gene including tac promoter region

| | | |
|---|---|---|
| (a-3) | 5'-CCAAAGTAAAGGAGAGTAGCACATAACGGTTCTGGCAAATATTC-3' | (SEQ ID NO: 16) |
| (b-3) | 5'-GCCATATGCGATACTCCTCCTCATTTCTTGCCCGCAGCGGCGCC-3' | (SEQ ID NO: 17) |

A sequence homologous to that of the DNA of the 5'-region of an *HPTL_0060* gene is added to primer (a-3), and a sequence required for ligation to a bleomycin resistance gene is added to the primer (b-3).

The resulting reaction mixture was subjected to gel electrophoresis. As a result, an about 0.4-kbp DNA fragment corresponding to the *rpsL* gene including *tac* promoter region was detected, and the DNA fragment was recovered from the gel.

### (3-2) Preparation of positive selection marker

The DNA fragment having a bleomycin resistance gene sequence was amplified by a PCR method through use of the DNA of *Streptoalloteichus hindustanus,* which had been artificially synthesized (its codon had been partially substituted) by using an approach known to a person skilled in the art, as a template. The following primers were used in the PCR.

| | | |
|---|---|---|
| (a-4) | 5'-GAAATGAGGAGGAGTATCGCATATGGCTAAACTTACTTCTGCTG-3' | (SEQ ID NO: 18) |
| (b-4) | 5'-CGGTGAAGCTCGAAACTCAATCTTGTTCCTCTGCAACAAAATG-3' | (SEQ ID NO: 19) |

A sequence required for ligation to the *rpsL* gene is added to primer (a-4), and a sequence homologous to that of the DNA of the 3'-adjacent region of the *HPTL_0060* gene is added to primer (b-4).

The resulting reaction mixture was subjected to gel electrophoresis. As a result, an about 0.4-kbp DNA fragment corresponding to the bleomycin resistance gene was detected, and the DNA fragment was recovered from the gel.

### (3-3) Preparation of marker cassette

An *rpsL* gene fragment including a tac promoter region and a bleomycin resistance gene fragment were mixed, and were linked and amplified by a PCR method. The following primers were used in the PCR.

| | | |
|---|---|---|
| (a-3) | 5'-CCAAAGTAAAGGAGAGTAGCACATAACGGTTCTGGCAAATATTC-3' | (SEQ ID NO: 20) |
| (b-4) | 5'-CGGTGAAGCTCGAAACTCAATCTTGTTCCTCTGCAACAAAATG-3' | (SEQ ID NO: 21) |

The resulting reaction mixture was subjected to gel electrophoresis. As a result, an about 1.0-kbp DNA fragment corresponding to a linked marker cassette gene was detected, and the DNA fragment was recovered from the gel.

### (4) Disruption of catechol 2,3-dioxygenase gene of host

### (4-1) Construction of DNA for disrupting HPTL_0060 gene

A DNA fragment corresponding to the 5'-side region of the *HPTL_0060* gene and a DNA fragment corresponding to the 3'-side region thereof were amplified by a PCR method through use of the genomic DNA of the wild strain of TH-1 strain as a template. The following primers were used in the PCR.

### Primers for amplifying 5'-side region of HPTL_0060 gene

| | | |
|---|---|---|
| (a-5) | 5'-CTACAACACGGATTCCTCTTTCCACTGGCAAG-3' | (SEQ ID NO: 22) |
| (b-5) | 5'-CTTCGGTGAAGCTCGAAACTGCTACTCTCCTTTACTTTGGGTGG-3' | (SEQ ID NO: 23) |

A sequence required for ligation to the 3'-side region gene is added to primer (b-5).

### Primers for amplifying 3'-side region of HPTL_0060 gene

| | | |
|---|---|---|
| (a-6) | 5'-GTAAAGGAGAGTAGCAGTTTCGAGCTTCACCGAAGTCTATAC-3' | (SEQ ID NO: 24) |
| (b-6) | 5'-GTTTTACAATTTAATGCAGATGTTCTTGAGTTCCGTGTAGAAC-3' | (SEQ ID NO: 25) |

A sequence required for ligation to the 5'-side region gene is added to primer (a-6).

The resulting reaction mixture was subjected to gel electrophoresis. As a result, an about 1.5-kbp DNA fragment and an about 2.0-kbp DNA fragment corresponding to the 5'-side region and 3'-side region of *HPTL_0060* gene, respectively were detected.

The DNA fragment of the 5'-side region of the *HPTL_0060* gene and the DNA fragment of the 3'-side region thereof thus prepared were mixed, and were linked and amplified by a PCR method. The following primers were used in the PCR.

| | | |
|---|---|---|
| (a-5) | 5'-CTACAACACGGATTCCTCTTTCCACTGGCAAG-3' | (SEQ ID NO: 22) |
| (b-6) | 5'-GTTTTACAATTTAATGCAGATGTTCTTGAGTTCCGTGTAGAAC-3' | (SEQ ID NO: 25) |

The resulting reaction mixture was subjected to gel electrophoresis. As a result, an about 3.5-kbp DNA fragment corresponding to the DNA fragment including the 5'-side region of *HPTL_0060* gene and the DNA fragment including the 3'-side region thereof, which had been linked, was detected, and the DNA fragment was recovered from the gel.

### (4-2) Construction of DNA for labeling HPTL_0060 gene

A DNA fragment corresponding to the 5'-side region of *HPTL_0060* gene and a DNA fragment corresponding to the 3'-side region thereof were amplified by a PCR method through use of the genomic DNA of TH-1 strain as a template. The following primers were used in the PCR.

### Primers for amplifying 5'-side region of HPTL_0060 gene

| | | |
|---|---|---|
| (a-5) | 5'-CTACAACACGGATTCCTCTTTCCACTGGCAAG-3' | (SEQ ID NO: 22) |
| (b-7) | 5'-CAGAACCGTTATGTGCTACTCTCCTTTACTTTGGGTGGATAAAC-3' | (SEQ ID NO: 26) |

A sequence required for ligation to the marker cassette is added to primer (b-7).

### Primers for amplifying 3'-side region of HPTL_0060 gene

| | | |
|---|---|---|
| (a-7) | 5'-GCAGAGGAACAAGATTGAGTTTCGAGCTTCACCGAAGTCTATAC-3' | (SEQ ID NO: 27) |
| (b-6) | 5'-GTTTTACAATTTAATGCAGATGTTCTTGAGTTCCGTGTAGAAC-3' | (SEQ ID NO: 25) |

A sequence required for ligation to the marker cassette is added to primer (a-7).

The resulting reaction mixture was subjected to gel electrophoresis. As a result, an about 1.5-kbp DNA fragment and an about 2.0-kbp DNA fragment corresponding to the 5'-side region and 3'-side region of *HPTL_0060* gene, respectively were detected, and the DNA fragments were recovered from the gel.

The DNA fragment of the 5'-side region of *HPTL_0060* gene and the DNA fragment of the 3'-side region thereof thus prepared, and the marker cassette prepared in the section (3-3) were mixed, and were linked and amplified by a PCR method. The following primers were used in the PCR.

| | | |
|---|---|---|
| (a-5) | 5'-CTACAACACGGATTCCTCTTTCCACTGGCAAG-3' | (SEQID NO: 22) |
| (b-6) | 5'-GTTTTACAATTTAATGCAGATGTTCTTGAGTTCCGTGTAGGAAC-3' | (SEQ ID NO: 25) |

The resulting reaction mixture was subjected to gel electrophoresis. As a result, an about 4.3-kbp DNA fragment corresponding to a DNA fragment including the DNA fragment of the 5'-side region, the DNA fragment of the 3'-side region, and a marker cassette DNA fragment was detected, and the DNA fragment was recovered from the gel. The DNA fragment is DNA for labeling *HPTL_0060* gene.

### (4-3) Labeling (positive selection) of HPTL 0060 gene of streptomycin-resistant strain

The streptomycin-resistant SR88 strain of TH-1 strain was transformed with the DNA for labeling *HPTL_0060* gene prepared in the section (4-2) by an electric pulse method (electroporation method), and was applied to LB medium containing 50 ug/mL of Zeocin serving as a bleomycin-based antibiotic, followed by culture at 52°C for 48 hours.

Each grown strain on the LB medium was restreaked onto LB medium containing 50 µg/mL of a bleomycin-based antibiotic Zeocin (product name), and was cultured at 52°C for 24 hours.

A DNA region including *HPTL_0060* gene was amplified by a PCR method through use of the genomic DNA of a Zeocin-resistant strain obtained as a result of the foregoing as a template. The following primers were used in the PCR. When the PCR is performed by using the combination of the following primers (a-3) and (b-8), in the case where *HPTL_0060* gene is substituted with the marker cassette, an about 1.2-kbp DNA region is amplified.

| | | |
|---|---|---|
| (a-3) | 5'-CCAAAGTAAAGGAGAGTAGCACATAACGGTTCTGGCAAATATTC-3' | (SEQ ID NO: 20) |
| (b-8) | 5'-CGGCGACGTTGATCCGTATCCCCAAATTCCGTG-3' | (SEQ ID NO: 28) |

The resulting reaction mixture was subjected to gel electrophoresis. As a result, an about 1.2-kbp DNA fragment corresponding to a sequence obtained by substituting the *HPTL_0060* gene with the marker cassette was detected. In the strain, the *HPTL_0060* gene is labeled with a marker.

### (4-4) Disruption (counterselection) of HPTL 0060 gene

The *HPTL_0060* gene-labeled strain obtained in the section (4-3) was transformed with the DNA fragment for disrupting the *HPTL_0060* gene obtained in the section (4-1) by electric pulse method (electroporation method), and was applied to an LB medium containing 400 µg/mL of streptomycin, followed by culture at 52°C for 48 hours.

Each grown strain on the LB medium was restreaked onto an LB medium containing 400 µg/mL of streptomycin, and was cultured at 52°C for 24 hours.

A DNA region including *HPTL_0060* gene was amplified by a PCR method through use of the genomic DNA of the streptomycin-resistant strain obtained as a result of the foregoing as a template.

The following primers were used in the PCR. When the PCR is performed by using the combination of the following primers (a-8) and (b-8), as long as homologous recombination with a DNA fragment for disrupting the *HPTL_0060* gene, which includes only a 5'-side region and a 3'-side region, and from which the *HPTL_0060* gene has been deleted, occurs, an about 0.3-kbp DNA region is amplified. The PCR was performed by an ordinary method through use of EmeraldAmp PCR Master Mix (manufactured by Takara Bio Inc.) as a reaction reagent.

| | | |
|---|---|---|
| (a-8) | 5'-GTGGTTGGAAAAATGAGCAAACCGTTTCTTGCCTTG-3' | (SEQ ID NO: 29) |
| (b-8) | 5'-CGGCGACGTTGATCCGTATCCCCAAATTCCGTG-3' | (SEQ ID NO: 28) |

The resulting reaction mixture was subjected to gel electrophoresis. As a result, an about 0.3-kbp DNA fragment, from which the *HPTL_0060* gene had been deleted, and which was amplified when being free of any marker cassette, was detected. That is, there were obtained strains, from each of which the *HPTL_0060* gene had been deleted, and in each of which the *HPTL_0060* gene had been disrupted. One of the strains was named Δ0060 strain.

### (5) Construction of expression plasmid of 3-dehydroshikimate dehydratase

The DNA fragment of *Pseudomonas thermotolerans-derived qsuB* (SEQ ID NO: 7) was amplified by a PCR.

### Primers for amplifying Pseudomonas thermotolerans qsuB

| | | |
|---|---|---|
| (a-9) | 5'-CTGGAGGAGAAACGCATATGCAGCGTTCGATCGCCACCGTCTC-3' | (SEQ ID NO: 30) |
| (b-9) | 5'-CGACGGAGCTCGAATTCTCAGAGCCTGGGCTGACGAGCGGCGC-3' | (SEQ ID NO: 31) |

A sequence homologous to that of vector pCAMO-6 is added to each of primers (a-9) and (b-9).

The resulting reaction mixture was subjected to gel electrophoresis. As a result, an about 1.9-kbp DNA fragment corresponding to the *qsuB* was detected, and the DNA fragment was recovered from the gel.

The DNA fragment of vector pCAMO-6 and the DNA fragment of the *qsuB* were ligated to each other to transform *Escherichia coli* JM109 strain. After that, plasmid extraction was performed to identify the sequence of the *qsuB* gene.

### (6) Construction of expression plasmid of protocatechuate decarboxylase

The DNA fragment of *Enterobacter asburiae*-derived *aroY* (SEQ ID NO: 3) was amplified by PCR.

### Primers for amplifying Enterobacter asburiae aroY

| | | |
|---|---|---|
| (a-10) | 5'-CAGATCTGGAGGAGAAACGCATATGGAAAACCCGATCAACGATC-3' | (SEQ ID NO: 32) |
| (b-10) | 5'-CTTGTCGACGGAGCTCGAATTCCTATTTCTTATCAGCGAACAGC-3' | (SEQ ID NO: 33) |

A sequence homologous to that of vector pCAMO-6 is added to each of primers (a-10) and (b-10).

The resulting reaction mixture was subjected to gel electrophoresis. As a result, an about 1.5-kbp DNA fragment corresponding to the *aroY* was detected, and the DNA fragment was recovered from the gel.

The DNA fragment of vector pCAMO-6 and the DNA fragment of the *aroY* were ligated to each other to transform *Escherichia coli* JM109 strain. After that, plasmid extraction was performed to identify the sequence of the *aroY* gene.

### (7) Construction of expression plasmid of catechol 1,2-dioxygenase

The DNA fragment of *Pseudomonas thermotolerans*-derived *catA* (SEQ ID NO: 5) was amplified by PCR.

### Primers for amplifying Pseudomonas thermotolerans catA

| | | |
|---|---|---|
| (a-11) | 5'-CTGGAGGAGAAACGCATATGACCGTGAAAATCACTCACACTG-3' | (SEQ ID NO: 34) |
| (b-11) | 5'-CGACGGAGCTCGAATTCTCAGGCGCCCACGACCTGCAGTGCACG-3' | (SEQ ID NO: 35) |

A sequence homologous to that of vector pCAMO-6 is added to each of primers (a-11) and (b-11).

The resulting reaction mixture was subjected to gel electrophoresis. As a result, an about 0.9-kbp DNA fragment corresponding to the *catA* was detected, and the DNA fragment was recovered from the gel.

The DNA fragment of vector pCAMO-6 and the DNA fragment of the *catA* were ligated to each other to transform *Escherichia coli* JM109 strain. After that, plasmid extraction was performed to identify the sequence of the *catA* gene.

### (8) Construction of plasmid for producing catechol

A plasmid for producing catechol was constructed by: linking the *qsuB* and the *aroY* using a ribosome binding sequence by a PCR method; and subjecting the resultant to seamless cloning.

The *Pseudomonas thermotolerans-derived qsuB* was amplified by PCR using the following primers through use of the expression vector obtained in the section (5) as a template.

| | | |
|---|---|---|
| (a-12) | 5'-GGCTCGTATAATGTGTGGAATTGTGAGCGGATAAC-3' | (SEQ ID NO: 36) |
| (b-12) | 5'-CATATGCGATACTCCTCCTCAGAGCCTGGGCTGACGAG CG-3' | (SEQ ID NO: 37) |

A sequence homologous to the sequence consisting of the ribosome binding sequence and the *aroY* sequence is added to the primer (b-12).

The resulting reaction mixture was subjected to gel electrophoresis. As a result, an about 1.9-kbp DNA fragment corresponding to the *qsuB* was detected, and the DNA fragment was recovered from the gel.

The *Enterobacter asburiae aroY* was amplified by a PCR using the following primers through use of the expression vector obtained in the section (6) as a template.

| | | |
|---|---|---|
| (a-13) | 5'-CTGAGGAGGAGTATCGCATATGGAAAACCCGATCAACGA TCTC-3' | (SEQ ID NO: 38) |
| (b-13) | 5'-CCGGCGGATTTGTCCTACTCAGGAGAGCGTTCACC-3' | (SEQ ID NO: 39) |

A sequence homologous to the ribosome binding sequence and that of the *qsuB* is added to primer (a-13).

The resulting reaction mixture was subjected to gel electrophoresis. As a result, an about 1.5-kbp DNA fragment corresponding to the *aroY* was detected, and the DNA fragment was recovered from the gel.

The DNA fragments of the *qsuB* and the *aroY* thus produced were mixed, and were linked and amplified with the following primers.

| | | |
|---|---|---|
| (a-12) | 5'-GGCTCGTATAATGTGTGGAATTGTGAGCGGATAAC-3' | (SEQ ID NO: 36) |
| (b-13) | 5'-CCGGCGGATTTGTCCTACTCAGGAGAGCGTTCACC-3' | (SEQ ID NO: 39) |

The resulting reaction mixture was subjected to gel electrophoresis. As a result, an about 3.5-kbp DNA fragment corresponding to a gene in which the *qsuB* and the *aroY* were linked was detected, and the DNA fragment was recovered from the gel.

The DNA fragment of vector pCAMO-6, and the gene in which the *qsuB* and the *aroY* were linked were ligated to each other to transform *Escherichia coli* JM109 strain. After that, plasmid extraction was performed to identify the sequences of the respective genes *qsuB* and *aroY*. The resultant plasmid is a plasmid for producing catechol.

### (9) Construction of plasmid for producing muconic acid

A plasmid for producing muconic acid was constructed by: linking the *qsuB*, the *aroY*, and the *catA* using a ribosome binding sequence by a PCR method; and subjecting the resultant to seamless cloning.

A DNA fragment in which the *qsuB* and the *aroY* were linked was amplified by using the plasmid for producing catechol produced in the section (8) as a template. The following primers were used in the PCR.

| | | |
|---|---|---|
| (a-12) | 5'-GGCTCGTATAATGTGTGGAATTGTGAGCGGATAAC-3' | (SEQ ID NO: 36) |
| (b-14) | 5'-ATGCGAGTCTCCTCCCTATTTCTTATCAGCGAACAGCTCCGGCG-3' | NO: 40) (SEQ ID |

A sequence homologous to the ribosome binding sequence is added to primer (b-14).

The resulting reaction mixture was subjected to gel electrophoresis. As a result, an about 3.5-kbp DNA fragment corresponding to the DNA in which the *qsuB* and the *aroY* were linked was detected, and the DNA fragment was recovered from the gel.

*Pseudomonas thermotolerans*-derived *catA* was amplified by a PCR using the following primers through use of the expression vector obtained in the section (7) as a template.

| | | |
|---|---|---|
| (a-14) | 5'-GAAATAGGGAGGAGACTCGCATATGACCGTGAAAATCA | CTCAC-3' (SEQ ID NO: 41) |
| (b-13) | 5'-CCGGCGGATTTGTCCTACTCAGGAGAGCGTTCACC-3' | (SEQ ID NO: 39) |

A sequence homologous to the ribosome binding sequence and that of the *aroY* is added to primer (a-14).

The resulting reaction mixture was subjected to gel electrophoresis. As a result, an about 1.5-kbp DNA fragment corresponding to the *catA* was detected, and the DNA fragment was recovered from the gel.

The DNA fragment in which the *qsuB* and the *aroY* were linked, and the *catA*-containing DNA fragment thus produced were mixed, and were linked and amplified by PCR using the following primers.

| | | |
|---|---|---|
| (a-12) | 5'-GGCTCGTATAATGTGTGGAATTGTGAGCGGATAAC-3' | (SEQ ID NO: 36) |
| (b-13) | 5'-CCGGCGGATTTGTCCTACTCAGGAGAGCGTTCACC-3' | (SEQ ID NO: 39) |

The resulting reaction mixture was subjected to gel electrophoresis. As a result, an about 4.5-kbp DNA fragment corresponding to a gene in which the *qsuB*, the *aroY*, and the *catA* were linked was detected.

The DNA fragment of vector pCAMO-6, and the DNA fragment in which the *qsuB*, the *aroY*, and the *catA* were linked were ligated to each other to transform *Escherichia coli* JM109 strain. After that, plasmid extraction was performed to identify the sequences of the *qsuB*, *aroY*, and *catA* genes.

### (10) Transformant of Hydrogenophilus thermoluteolus

### (10-1) Introduction of gene into wild strain

*Hydrogenophilus thermoluteolus* TH-1 strain was transformed with each of the *qsuB*-containing plasmid obtained in the section (5), the *qsuB*- and *aroY*-containing plasmid obtained in the section (8), and the *qsuB*-, *aroY*-, and *catA*-containing plasmid obtained in the section (9) by electric pulse method (electroporation method), and was applied to solid LB medium containing 50 µg/mL of kanamycin, followed by culture at 52°C for 24 hours. In addition, TH-1 strain was similarly transformed with pCAMO-6. Each grown strain on the solid LB medium was inoculated with a platinum loop into solid LB medium containing 50 µg/mL of kanamycin, and was cultured at 52°C for 24 hours. The amplification of the inserted fragment of each plasmid was recognized for each grown strain on the solid LB medium by a PCR.

As a result, the amplification of a DNA fragment having a length corresponding to each gene was recognized. The respective plasmids and TH-1 strain transformants transformed with the plasmids were named as shown in Table 1.

The plasmid-introduced strains prepared as described above were inoculated with a platinum loop into a liquid A medium containing 50 µg/mL of kanamycin, and a gas mixture of H₂:O₂:CO₂ = 7.5:1:1.5 was supplied along with their culture, followed by shaking culture at 52°C for 72 hours. After the culture, the cultured product was centrifuged (4°C, 5,000 g, 10 minutes) to provide a culture supernatant.

### (10-2) Introduction of gene into HPTL_0060 gene-disrupted strain (Δ0060 strain)

Δ0060 strain was transformed with each of the *qsuB*-containing plasmid obtained in the section (5), the *qsuB*- and *aroY*-containing plasmid obtained in the section (8), and the *qsuB*-, *aroY*-, and *catA-*containing plasmid obtained in the section (9) by electric pulse method (electroporation method), and was applied to solid LB medium containing 100 µg/mL of kanamycin and 400 µg/mL of streptomycin, followed by culture at 52°C for 24 hours. Each grown strain on the solid LB medium was inoculated with a platinum loop into solid LB medium containing 100 µg/mL of kanamycin and 400 µg/mL of streptomycin, and was cultured at 52°C for 24 hours. In addition, Δ0060 strain was similarly transformed with pCAMO-6. The amplification of the inserted fragment of each plasmid was recognized for each grown strain on the solid LB medium by PCR.

As a result, the amplification of a DNA fragment having a length corresponding to each gene was recognized. The respective plasmids and Δ0060 strain transformants transformed with the plasmids were named as shown in Table 1.

The plasmid-introduced strains prepared as described above were inoculated with a platinum loop into liquid A medium containing 100 µg/mL of kanamycin and 400 µg/mL of streptomycin, and a gas mixture of H₂:O₂:CO₂ = 7.5:1:1.5 was supplied along with their culture, followed by shaking culture at 52°C for 72 hours. After the culture, the cultured product was centrifuged (4°C, 5,000 g, 10 minutes) to provide a culture supernatant.

### (10-3) Production of catechol and protocatechuic acid

The concentrations of catechol and protocatechuic acid in the culture supernatant were measured with a high performance liquid chromatograph (Shimadzu Corporation) under the following conditions.

| | |
|---|---|
| Mobile phase A: | 0.1% phosphoric acid water |
| Mobile phase B: | 50% acetonitrile |
| Flow rate: | 1 mL/min |
| Column: | CAPCELLPAK MGII, 5.0 µm, 4.6 mm |
| I.D.×150 mm | |
| Column temperature: | 40°C |
| PDA temperature: | 40°C |
| PDA: | from 200 nm to 300 nm |
| Reference wavelength: | 210 nm |

### (10-4) Production of muconic acid

The concentration of muconic acid in the culture supernatant was measured with a high performance liquid chromatograph (Shimadzu Corporation) under the following conditions.

| | |
|---|---|
| Mobile phase A: | 5 mmol/L p-toluenesulfonic |
| acid: | ethanol 90:10 (v/v) |
| Mobile phase B: | 5 mmol/L p-toluenesulfonic acid, 20 |
| mmol/L Bis-Tris, 0.1 mmol/LEDTA: | ethanol 90:10 (v/v) |
| Flow rate: | 0.7 mL/min in each of the mobile phases A and B |
| Column: | Shim-pack SCR-102H |
| Column temperature: | 45°C |
| Detector: | CDD-10A VP |
| Sample injection amount: | 20 µL |

The results are shown in Table 1. In each of TH-1 strain having introduced thereinto the *qsuB* and Δ0060 strain having introduced thereinto the *qsuB*, protocatechuic acid was detected in the culture supernatant. Meanwhile, when the *qsuB* and the *aroY*, or the *qsuB*, the *aroY*, and the *catA* were introduced, in the case where the host was TH-1 strain, catechol and muconic acid were not detected in the culture supernatant, and only in the case where the host was Δ0060 strain, catechol was, or catechol and muconic acid were, detected in the culture supernatant.

It was concluded from the foregoing that the *HPTL_0060* gene was a catechol 2,3-dioxygenase gene, and in a *Hydrogenophilus* bacterium, the disruption of the *HPTL_0060* gene was essential to efficient production of muconic acid or catechol.

**[Table 1]**

| Bacterial strain | Host | Introduced gene | Protocatechuic acid concentration (mM) | Catechol concentration (mM) | Muconic acid concentration (mM) |
|---|---|---|---|---|---|
| pCAMO-6/TH-1 | Wild type (TH-1 strain) | pCAMO-6 | ND | ND | ND |
| pCAMO-6/Δ0060 | Δ0060 strain | pCAMO-6 | ND | ND | ND |
| PAl01 | Wild type (TH-1 strain) | *qsuB* | 1.5 | ND | ND |
| PAl01 / Δ0060 | Δ0060 strain | *qsuB* | 1.5 | ND | ND |
| CAl01 | Wild type (TH-1 strain) | *qsuB, aroY* | ND | ND | ND |
| CAl01/Δ0060 | Δ0060 strain | *qsuB, aroY* | ND | 1.0 | ND |
| MAl01 | Wild type (TH-1 strain) | *qsuB, aroY, catA* | ND | ND | ND |
| MAl01/Δ0060 | Δ0060 strain | *qsuB, aroY, catA* | ND | 0.35 | 0.9 |

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1 to 11 are shown below.
[Chem. 3]
   SEQ ID NO: 1 (Catechol 2,3-dioxygenase gene of *Hydrogenophilus thermoluteolus*) 
atgggcatcctgcgtattggtcacgctagcctgcgcgtcatggacatggccgcggcggtgcgccactatgaaaa
cgttctcggcatgaaaaggtgatggaagacaagcacggaacgtctacctcaaatgctggacgaatggaca
agttctccctcatcctgaccccttccgatcgagcgggcctcaaccatgtcgcctacaaggtagaacgggaagag
gatctcgacgagctccaagaaaagatcgaggcctatggcatctccacgacaatgctccccgaaggacgttgcc
ctccactggccgcatgttgcaattcaacctcccaagcggccacgaaatgcgtctctacgcgttcaaagagtgcg
tgggcactgatgtcggcaacatcaaccctgacccgtggccggacgatattcgcggcgccggggctcactggttg
gatcattgccttttgatgtgtgagctcaaccccgaaaccggagtcaataaagtcgcggaaaacaccaaatttgc
cacagaagtgctcggcttttcctgaccgaacaagttgtggtcggaccgaacggttcgattcaggcggccacct
ggttggcacgtaccacaacgccgcacgatatcgcctttgttggcggaccgcgcatggggctccaccacattgcg
ttcttcctcgacggatggaacgatatcctcaaagcggccgacgtgatggcaaagaacaaagtgaagatcgacgt
ggcgccaacgcggcatggcatcacgcgggggaaaccatctacttctttgatcccagcggcaaccgcaacgaaa
cgttcgctgggctcggttaccttgcccaacccgatcggcccgttgttacctggaccgaagagcacctaggcagc
ggcatctttaccacactggggagctcgttcgagcttcaccgaagtctatacctaa
[Chem. 4]
   SEQ ID NO: 2 (Catechol 2,3-dioxygenase of *Hydrogenophilus thermoluteolus*) 
MGTLRIGHASLRVMDMAAAVRHYENVLGMKKVMEDKHGNVYLKCWDEWDKFSLILTPSDRAGLNHVAYKVEREE
DLDELQEKIEAYGISTTMLPEGTLPSTGRMLQFNLPSGHEMRLYAFKECVGTDVGNINPDPWPDDIRGAGAHWL
DHCLLMCELNPETGVNKVAENTKFATEVLGFFLTEQVVVGPNGSIQAATWLARTTTPHDIAFVGGPRMGLHHIA
FFLDGWNDILKAADVMAKNKVKIDVAPTRHGITRGETIYFFDPSGNRNETFAGLGYLAQPDRPVVTWTEEHLGS
GIFYHTGELVSSFTEVYT
[Chem. 5]
   SEQ ID NO: 3 (Protocatechuate decarboxylase gene of *Enterobacter asburiae*) 
atggaaaacccgatcaacgatctccgcagcgccatcgccctgcttcagcgccacgaagggcagtatcttgaaac
cgattatccggtcgatcccaacgcggagctggcggggtttaccgccatatcggcgcggcggcacggtaaaac
gtcccacccgtacggaccggccatgatgttcaacagcgtgaagggctatcctggctcccgcatcctggtgggc
atgcacgccagccgcgagcgcgcggcgctgcttctgggctgcgagccctctgagctggcaaaacacgtcggtca
ggcggtgaaaaagccggttgcgccggttgtcgttccggcctcgcaggcgccctgccaggaacaagttttctacg
cggacgaccctgacttcgacctgcgcaagctgcttccggccccaacaaacacgccgattgatgccggtccgttc
ttctgtctggggctggtgctggcaagcgatccggaagacagctcgctgacggatgtcaccattcaccgcctctg
cgttcaggagcgcgacgagctttccatgttccttgccgccggtcggcatatcgaagtcttccgcaaaaaggcgg
aagaggccggaaaaccgctgccggtaacgatcaacatggggctcgatcccgccatctatatcggcgcatgttt
gaggcgcccaccacgccgtttggctacaacgagctgggcgtcgccggggcgctgcgccagcagcctgtcgaact
ggtgcagggtgtggcggtaaaagagaaagcgatcgcgcggcggaaatcatcatcgagggcgaactgctccctg
gcgtgcgggtaagagaagatcagcacaccaataccggccacgcgatgccggagttcccgggctactgcggcgag
gcgaacccgtccctgccggtgatcaaagtgaaagccgtgaccatgcgaaaccacgcgatcctgcaaacgctggt
gggaccgggagaagaacataccacgctggccggtttgccgacggaggccagcatccgcaatgccgttgaagagg
cgatcccgggcttcctgcaaaacgtctatgcgcataccgcaggcggcggtaagttcctcgggatattgcaggtg
aaaaaacgccagccgtcagacgaaggtcgtcagggccaggcggcgctgattgccttagccacttactcggagct
gaagaacatcattcttgtcgatgaagacgtggatatttcgacagcgacgacatcctgtgggcgatgaccaccc
gcatgcagggtgatgtcagcatcacgaatattccggggatccgcggccaccagctggatccgtcccagtcgccg
gattacagcacctcgattcgcgcaaacggcatctcctgcaagaccatcttcgactgcacggtgccgtgggcgct
gaaggaccgcttcgagcgagcgccgtttatggaggtggatccgcgtccgtgggcgccggagctgttcgctgata
agaaatag
[Chem. 6]
   SEQ ID NO: 4 (Protocatechuate decarboxylase of *Enterobacter asburiae*) 
MENPINDLRSAIALLQRHEGQYLETDYPVDPNAELAGVYRHIGAGGTVKRPTRTGPAMMFNSVKGYPGSRILVG
MHASRERAALLLGCEPSELAKHVGQAVKKPVAPVVVPASQAPCQEVFYADDPDFDLRKLLPAPTNTPIDAGPF
FCLGLVLASDPEDSSLTDVTIHRLCVQERDELSMFLAAGRHIEVFRKKAEEAGKPLPVTINMGLDPAIYIGACF
EAPTTPFGYNELGVAGALRQQPVELVQGVAVKEKAIARAEIIIEGELLPGVRVREDQHTNTGHAMPEFPGYCGE
ANPSLPVIKVKAVTMRNHAILQTLVGPGEEHTTLAGLPTEASIRNAVEEAIPGFLQNVYAHTAGGGKFLGILQV
KKRQPSDEGRQGQAALIALATYSELKNIILVDEDVDIFDSDDILWAMTTRMQGDVSITNIPGIRGHQLDPSQSP
DYSTSIRANGISCKTIFDCTVPWALKDRFERAPFMEVDPRPWAPELFADKK
[Chem. 7]
   SEQ ID NO: 5 (Catechol 1,2-dioxygenase gene of *Pseudomonas thermotolerans*) 
atgaccgtgaaaatcactcacactgctgaaatccagaagttcttcgaggaagccgccggtttcggcaacgacaa
cggcagcccgcgcctgaagcgcatcatgctgcgcatcctgcaggacaccgccaagctgatcgaggacctggaaa
tcacccaggacgagttctggttcgccgtggactacatcaaccgtctcggcgcccgcggcgaagccggcctgctg
gtcgccggcctgggcctggagcacttcctcgacctgctgcaggacgccaaggacgagcagctgggtcgtaccgg
tggtaccccgcgcaccatcgaaggtccgctgtacgtggccggcgcgcccatcgccgaaggcgaggcccgcatgg
acgacggcagcgaagaggacgtcgccaccgtgatgttcctggaaggccgcgtgctggatctggacggcaagccg
atcgccggcgccaccgtggatctgtggcacgccaacaccaagggcaactactcctacttcgacaagagccagag
cgagtacaacctgcgtcgccgcatcatcaccgatgccgaaggtcgctaccgcgcccgcagcatcgtgccgtccg
gctacggctgcgacccgcagggcccgacccaggagtgcctggacctgctcggccggcatggccagcgcccggcg
cacatccacttcttcatctccgcgcctggctaccggcacctgaccacccagatcaacctggccggcgaccgtta
cctgtgggacgacttcgcctacgccacccgcgacggcctggtgggcgacatccgcttcatcgaggatgccgaag
ccgccgccaagcgtggcgtgaagggccgcttcgccgagctggagttcaacttccagctgcagaaggccagcagc
gccgaagacgagcagcgcagccgccgtccccgtgcactgcaggtcgtgggcgcctga
[Chem. 8]
   SEQ ID NO: 6 (Catechol 1,2-dioxygenase of *Pseudomonas thermotolerans*) 
MTVKITHTAEIQKFFEEAAGFGNDNGSPRLKRIMLRILQDTAKLIEDLEITQDEFWFAVDYINRLGARGEAGLL
VAGLGLEHFLDLLQDAKDEQLGRTGGTPRTIEGPLYVAGAPIAEGEARMDDGSEEDVATVMFLEGRVLDLDGKP
IAGATVDLWHANTKGNYSYFDKSQSEYNLRRRIITDAEGRYRARSIVPSGYGCDPQGPTQECLDLLGRHGQRPA
HIHFFISAPGYRHLTTQINLAGDRYLWDDFAYATRDGLVGDIRFIEDAEAAAKRGVKGRFAELEFNFQLQKASS
AEDEQRSRRPRALQVVGA
[Chem. 9]
   SEQ ID NO: 7 (3-Dehydroshikimate dehydratase gene of *Pseudomonas thermotolerans*) 
atgcagcgttcgatcgccaccgtctccctgagcggtacacttccggaaaaactcgaagccatagccgccgccgg
attcgacggcgtggagatattcgagaacgatctgctctactacgccggcagtccccgcaagtgcgtcagctat
gcgccgacctcggcctggccatcacgctgttccagccgtttcgcgacttcgagggctgccgtcgcgaccgcctg
gagaagaatctcgaccgcgccgagcgcaagttcgacctgatgcaggagctgggcaccgacctgattctggtgtg
cagcaacgtggcaccggattccctgggcgatccgcgtatcatcgccgacgacctgcagcgcctcgcagagcggg
ccggcgctcgcggcctgcgcatcggctacgaggccctggcctggggccgccacgtgaacagctggcagcaggtt
tggaacatcgttcgcgaggtggaccaccccgccctcggcgtgttgctggacagcttccacaccctgtcgatcca
gggcgacccgagcggcatcgccgagattcccggcgacaggatcttcttcgtacagatggccgacgcgcccatcc
tggccatggatgtgctggagtggagccgccacttccgctgcttccccggacagggcgagttcgatctggccggc
ttcctggcgccgatcctgaagagcggctaccgcggcccgctgtccctggagatcttcaatgacggcttccgcgc
cgcgccgccgcgttccaatgccgccgacggcctgcgctcgctgctctacctggaggagaaactcggcagcgcc
tggccgcggagggtagtccgctggccgagtcggagcaactgttcgcaccgccgcctgccagcccctacgagggc
gtggagttcctcgagttcgccgtggacgaggagcagggtgcgcgcctggctagctggctggagaaactcggttt
cgcccgcgtcggccgacaccgttccaaggaggtcagcctgctgcgccaggggatatcaacctggtgctcaacg
ccgagccctattccttcgcccgcaacttcttcgaggcccatggtccctcgttgtgcgccaccgcgctgcgggtt
cgcgaccaggctggcgccctggcccgcgcccagtccttcagggccagccgttccgcggcctggtcggtcccaa
cgagaccgagatccccgcggtgcgtgccccggacggcagcctgatctatctggtggagcgcagtgcaccggggc
agaccatctacgacaaggacttcctgctcgacgggcaggcccggcccagcggcagcctcaagcgcatcgaccac
atggccgtggcgctgccggccgacagcctggatagctgggtgttgttctacaagagcctgttcgacttcgaggc
cgacgacgaggtggtgttacctgacccctacggcctggtgaagagccgggccatccgcagccgctgcagcaccg
tgcgcctgccgctgaacatctcggagaaccgcaacacggcgatctcccacgcgctgtccagctaccgcggctcc
ggcgtgcaccacatcgccttctcctgcgaggacctgttcgccgaggtgagccgcgccaaggacgccggagtgcc
gctgctggagatcccgctcaactactacgacgacctggccgcgcgtttcgacttcgacgacgaattcctcagcg
agctggcctactacaacgtgctctacgatcgcgatgcccagggtggcgagctgttccacgtctacaccgagcct
ttcgaaggacgcttcttcttcgagctgctgcagcggcgcaacggctataccggctacggtgcggccaacgtcgc
cgtacgtctggccgcgctggccaagacgcgcagcggcgccgctcgtcagcccaggctctga
[Chem. 10]
   SEQ ID NO: 8 (3-Dehydroshikimate dehydratase of *Pseudomonas thermotolerans*) 
MQRSIATVSLSGTLPEKLEAIAAAGFDGVEIFENDLLYYAGSPREVRQLCADLGLAITLFQPFRDFEGCRRDRL
EKNLDRAERKFDLMQELGTDLILVCSNVAPDSLGDPRIIADDLQRLAERAGARGLRIGYEALAWGRHVNSWQQV
WNIVREVDHPALGVLLDSFHTLSIQGDPSGIAEIPGDRIFFVQMADAPILAMDVLEWSRHFRCFPGQGEFDLAG
FLAPILKSGYRGPLSLEIFNDGFRAAPPRSNAADGLRSLLYLEEKTRQRLAAEGSPLAESEQLFAPPPASPYEG
VEFLEFAVDEEQGARLASWLEKLGFARVGRHRSKEVSLLRQGDINLVLNAEPYSFARNFFEAHGPSLCATALRV
RDQAGALARAQSFRGQPFRGLVGPNETEIPAVRAPDGSLIYLVERSAPGQTIYDKDFLLDGQARPSGSLKRIDH
MAVALPADSLDSWVLFYKSLFDFEADDEVVLPDPYGLVKSRAIRSRCSTVRLPLNISENRNTAISHALSSYRGS
GVHHIAFSCEDLFAEVSRAKDAGVPLLEIPLNYYDDLAARFDFDDEFLSELAYYNVLYDRDAQGGELFHVYTEP
FEGRFFFELLQRRNGYTGYGAANVAVRLAALAKTRSGAARQPRL
[Chem. 11]
   SEQ ID NO: 9 (pCAMO-6) Former half 
CCCATCGACTGCACGGTGCACCAATGCTTCTGGCGTCAGGCAGCCATCGGAAGCTGTGGTATGGCTGTGCAGGT
CGTAAATCACTGCATAATTCGTGTCGCTCAAGGCGCACTCCCGTTCTGGATAATGTTTTTTGCGCCGACATCAT
AACGGTTCTGGCAAATATTCTGAAATGAGCTGTTGACAATTAATCATCGGCTCGTATAATGTGTGGAATTGTGA
GCGGATAACAATTTCACACAGGAAACAGGCCAGTTAGGCCTGCTAGCAGATCTGGAGGAGAAACGCATATGCTG
ATATCGGATCCGAATTCGAGCTCCGTCGACAAGCTTGCGGCCGCACTGCAGCACCATCACCACCATCATTGATA
AGATCCGGCTGCTAACAAAGCCCGAAAGGAAGCTGAGTTGGCTGCTGCCACCGCTGAGCAATAACTCGAGGCCA
ATGAGGCCTTCAAATAAAACGAAAGGCTCAGTCGAAAGACTGGGCCTTTCGTTTTATCTGTTGTTTGTCGGTGA
ACGCTCTCCTGAGTAGGACAAATCCGCCGGGAGCGGATTTGAACGTTGCGAAGCAACGGCCCGGAGGGTGGCGG
GCAGGACGCCCGCCATAAACTGCCAGGCATCAAATTAAGCAGAAGGCCATCCTGACGGATGGCCTTTTTGCGTT
TCTACAAACTCCGCGGATCTAGGTGAAGATCCTTTTTGATAATCTCATGACCAAAATCCCTTAACGTGAGTTTT
CGTTCCACTGAGCGTCAGACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTCTAGTGTAGCCGTAGTTAGGCCACC
ACTTCAAGAACTCTGTAGCACCGCCTACATACCTCGCTCTGCTAATCCTGTTACCAGTGGCTGCTGCCAGTGGC
GATAAGTCGTGTCTTACCGGGTTGGACTCAAGACGATAGTTACCGGATAAGGCGCAGCGGTCGGGCTGAACGGG
GGGTTCGTGCACACAGCCCAGCTTGGAGCGAACGACCTACACCGAACTGAGATACCTACAGCGTGAGCTATGAG
AAAGCGCCACGCTTCCCGAAGGGAGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGCAACGCGGCCTTTTTACGGTTCC
TGGCCTTTTGCTGGCCGCGGACGTAGAAAGCCAGTCCGCAGAAACGGTGCTGACCCCGGATGAATGTCAGCTAC
TGGGCTATCTGGACAAGGGAAAACGCAAGCGCAAAGAGAAAGCAGGTAGCTTGCAGTGGGCTTACATGGCGATA
GCTAGACTGGGCGGTTTTATGGACAGCAAGCGAACCGGAATTGCCAGCTGGGGCGCCCTCTGGTAAGGTTGGGA
AGCCCTGCAAAGTAAACTGGATGGCTTTCTTGCCGCCAAGGATCTGATGGCGCAGGGGATCAAGATCCGATCAA
GAGAGGCCACTAAGGCCCAGGATGAGGATCGTTTCGCATGATTGAACAAGATGGATTGCACGCAGGTTCTCCGG
CCGCTTGGGTGGAGAGGCTATTCGGCTATGACTGGGCACAACAGACAATCGGCTGCTCTGATGCCGCCGTGTTC
CGGCTGTCAGCGCAGGGGCGCCCGGTTCTTTTTGTCAAGACCGACCTGTCCGGTGCCCTGAATGAACTACAGGA
CGAGGCAGCGCGGCTATCGTGGCTGGCCACGACGGGCGTTCCTTGCGCAGCTGTGCTCGACGTTGTCACTGAAG
CGGGAAGGGACTGGCTGCTATTGGGCGAAGTGCCGGGGCAGGATCTCCTGTCATCTCACCTTGCTCCTGCCGAG
AAAGTATCCATCATGGCTGATGCAATGCGGCGGCTGCATACGCTTGATCCGGCTACCTGCCCATTCGACCACCA
AGCGAAACATCGCATCGAGCGAGCACGTACTCGGATGGAAGCCGGTCTTGTCGATCAGGATGATCTGGACGAAG
AGCATCAGGGGCTCGCGCCAGCCGAACTGTTCGCCAGGCTCAAGGCGCGTATGCCCGACGGCGAGGATCTCGTC
GTGACTCATGGCGATGCCTGCTTGCCGAATATCATGGTGGAAAATGGCCGCTTTTCTGGATTCATCGACTGTGG
CCGGCTGGGTGTGGCGGACCGCTATCAGGACATAGCGTTGGCTACCCGTGATATTGCTGAAGAGCTTGGCGGCG
AATGGGCTGACCGCTTCCTCGTGCTTTACGGTATCGCCGCTCCCGATTCGCAGCGCATCGCCTTCTATCGCCTT
CTTGACGAGTTCTTCTGAGGCCATCTAGGCCATGAGTAAGTCTGACGGAACGGAAAGTTGTCGGTTACCAGCAA
CGTTTCGCCCTGTTGGCCTCAAAAGTTCGAGTCCGTTATTGGTATTTCCATACCAACTCAAACGGGATATAATG
TGACATGTAACATTTCTCGATTGAGGAACGACAATGACAGTACCGGTCAAAGCGCGTGTTCAAAAGTATCGCGA
CGCGCTGCGCCGCTCGGGTTTGCGTCCGGTTCAGATATGGGTGCCGGATACCCGCCGCCCGGGCTTTGCACAAG
[Chem. 12]
   SEQ ID NO: 9 (pCAMO-6) Latter half 
AGTGTCGCCGCCAGTGCCTGCTCGTGACCCAGGCAGACGCCGCTGATACGCAACTCCAACGACTCATGGATGAA
GCCTTGGCAGAGATTGATGGCTGGCAGGCATGATGCGCGGCGATTTGGTAACGATCGTTACCCAGGGCGACTTT
GGCAAACCAAGGCCAGCGTTGGTGATCCAGTCCGATCTGTTCAACGAGCTCAGCAGCATAACCGTCTTTCCTGT
AACCAGTACCATTGTGGATGCCCCGTTGTTGCGCGTCACGGTTGAGCCAACCCCGGAAAACGGCTTGCACAAGC
GATCACAGGTGATGGTGGACAAAATTATGACCGTGCGGCGGGACAAAGTCGGTCAGATCATTGGACGTATCGAT
ACGACTACGCTGACAACGATCGAGCGCTGCTTAGCTGTCTTCTTGGGAATCGCGAAATAAGGCCATGAAGGCCT
TACCCATCACCCTGCATCACGCCGACCGCTTCGCCACTTTGCGACAAGAAAGCAATCTGAACGCGCTGGCGAAA
ATGGATCGGCGCTGCCCGGTCTACCTGTGAAGTTCGCGCAAACGTCCATCGAGACGTACATTGCCGTTGGCATC
GATCTCGACGGCGATTTCCTCTGGCTACCGATCGCGCGCACCGCTTACGCGCCCTCGACGTAGTCGAGAAAAGC
GTGATTGCGTATGTTGCGCCAACTGAGCCCGTCGGTGCGTTGTGACCACTCGTTGTAGACCACGCCCAAAACCA
TCACCCCAAACGGTGCGGAAAGTGCTACTCTTTCGTCTTGCCACAACAAGAAGGAGGGCGGGTGGTGATCGAAA
CCGGTGCTTTGAGAGCCAAAGAAGACCCCATTGCCTGGGTCCAACAGAAAGCAGCCGAGGCGTTGCAACGCCTA
GATCAAAAGATGGCCTCTCAGCCCGTGCAGATGTACCTACCGGGGTTCGATCTAGGGGCCTTTCCCAACCATGT
GAACCGCTCCTCGCTCTTTGCGCCTATTGCGCGCGGTCGCCGCAAATTTCATCGACAAACGAAGATGGTCAGCC
GCTCGGATGCTGTGATCGAATACACTGGAGAGCAGCTCGACGAAGCCGACTGCGACCTCATGATGGCGCTCATC
TGGTTTGCCCAGAAAAAACCGTTAGGGGAGTGGGTGACATTGAACCGAGCGCAGCTGTTGCGGCGGATCGGGCG
CGCGACGGGCAAGCAGAACTACGAGTGGCTCCATCGCAGAATGCGGGCGATGACCGAAGCGACACTCTACATCG
AAGTGCGCAAATCCGACGGCAGCACACGCTACAAGATCGGCTACGCCGAGGCGTTTCACCTCATCAGCGGCTTT
GCGTTCGATGAGAAAAGCGACGAATACTGCTACCAACTCGATCCCCGCTGGGTACAGCTCTTCAGCAACCGCGA
ATACAGCCTGATCAACTTCGACTTGCGATTGCAGATCAAACGGGGTCAGGACATGGCGAAAACCCTGCAACGCC
TCGTTGCTACTTCTTCCGACCCCATCCAGTCTTACGAACTCGAATGGCTCAAGAGCAAAATGGAATACACCGGA
AGAATGCGCGATTTCAAAGAGTCGCTCTCCAAGGCGTGCAATGAGCTGAAACGGTTGCACATCGTCAGTGAATA
CAAGATCACTCTGGACAAAAAGAAGCAACGGAGCTGTTGAAGCTCTGGATTCCCAAAGCGATCACGGCATAAA
AGCGGTGCGACACTGCACGATCAGAAAGTATTTTCAGTTACTCAAACACCGTTTTTTGTTGAAAAACAACGAAT
TGTCATCGGTATAGCGTCGCGCACCCGAGACGACCGACGGTGTCTGGCCATCGGTATAGCGTCGCGCTTTCTCC
ACCTCTTGAACAGTCGTTCGGCATCGGTATAGCGTCGCGCCTGCATCGGTATAGCGTCGCGCTTTTTCGGTATA
GCGTCGCGCTTTTTCGGTATAGCGTCGCGCGGTTGTCAAAAAATCTATTATTATTCAATAAGTTACAAGCGGTT
TTTGACCGTCTACTATATTTCTACTTATTTCTACTAGGAAGCGCCTGTGGATAACTTCGCTCTTTTCTACACAG
CCCCTCAGCGGCGCAAGCGCGCCGCTGGGTAGAAAATTCCTATCGCAACGAACTGTCGTTCCTAAAAATAGGCT
ACTCGGTAAAACAACCGTATAAATGATGCCGCGTGCTCGGAGTCAACTCCAGAACGGCATCGGTATAGTGTCGC
ACTTAGCAATGTGCCGAATATTTCATCGTTGAGCCGACTTTTGGAGATTGGTCATGGGAGATCCTCGGGTTGTCG
AAGCCTTGGACTCCTGTTGATAGATCCAGTAATGACCTCAGAACTCCATCTGGATTTGTTCAGAACGCTCGGTT
GCCGCCGGGCGTTTTTTATTGGTGAGAATCCAGTCTAGA
[Chem. 13]
   SEQ ID NO: 10 (Gene responsible for streptomycin sensitivity of *Hydrogenophilus thermoluteolus*) 
atgccaaccatcaaccagttggtgcgtcgtccgcggaaaacggcgcccgaaaagagcaaagtgccggcgttgca
gggatgtccgcaaaaacgaggcgtgtgtacgcgcgtctataccacgacgccgaaaaagccgaactcggcccttc
gtaaggtcgcgaaagtgcgtttgaccaacggttacgaggtgatttcgtacatcggcggcgaaggcacaatctg
caagaacactcggtggtgctgattcgtggcggccgggtgaaagacctgccgggtgtgcgttaccacatcgtgcg
cggttcgctcgacttgcaaggggtcaaggaccgtaagcaagggcgttccaagtacggggcgaagcgtccgaagc
cgggcgccgctgcgggcaagaaataa
[Chem. 14]
   SEQ ID NO: 11 (Bleomycin resistance gene of *Streptoalloteichus hindustanus*)

atggctaaacttacgtccgctgttccagttcttacggctcgtgatgttgcagaagctgttgaatttggacgga
tcgtttgggttttcccgtgttttcgtggaagacgattttgcaggtgttgttcgtgatgatgttacgcttttta
tttccgctgtgcaagatcaagttgttccagataatacgcaagcatgggtttgggttcgtggtttggatgagctt
tatgctgaatggtccgaagttgtgtccacgaatttcgtgatgcatccggtccagctatgacggagattgttga
acaaccatggggtcgtgaatttgcacttcgtgatccagctggtaattgcgttcatttgttgcagaggaacaag
attga

### Industrial Applicability

The transformant of the present invention can highly efficiently produce muconic acid or catechol using carbon dioxide as a sole carbon source, thus providing a solution to global warming caused by increased carbon dioxide emissions and contributing to high-efficient industrial production of chemical products.

## Claims

1. A *Hydrogenophilus* bacterium gene-disrupted strain, comprising a disrupted catechol 2,3-dioxygenase gene.

2. The *Hydrogenophilus* bacterium gene-disrupted strain according to claim 1, wherein the catechol 2,3-dioxygenase gene is any one of the following DNAs (a1) to (a5).
(a1) A DNA comprising the nucleotide sequence of SEQ ID NO: 1
(a2) A DNA comprising a nucleotide sequence that has 90% or more identity to SEQ ID NO: 1 and encoding a polypeptide having catechol 2,3-dioxygenase activity
(a3) A DNA encoding a polypeptide comprising the amino acid sequence of SEQ ID NO: 2
(a4) A DNA encoding a polypeptide comprising an amino acid sequence that has 90% or more identity to SEQ ID NO: 2, the polypeptide having catechol 2,3-dioxygenase activity
(a5) A DNA encoding a polypeptide comprising an amino acid sequence that has 1 to 30 amino acids deleted, substituted, inserted, or added in the amino acid sequence of SEQ ID NO: 2, the polypeptide having catechol 2,3-dioxygenase activity

3. A *Hydrogenophilus* bacterium transformant, in which a catechol 2,3-dioxygenase gene on its genome is disrupted, comprising (i) a 3-dehydroshikimate dehydratase gene and a protocatechuate decarboxylase gene that are exogenous, or (ii) a 3-dehydroshikimate dehydratase gene, a protocatechuate decarboxylase gene, and a catechol 1,2-dioxygenase gene that are exogenous.

4. The *Hydrogenophilus* bacterium transformant according to claim 3, wherein the protocatechuate decarboxylase gene is any one of the following DNAs (b1) to (b5).
(b1) A DNA comprising the nucleotide sequence of SEQ ID NO: 3
(b2) A DNA comprising a nucleotide sequence that has 90% or more identity to SEQ ID NO: 3 and encoding a polypeptide having protocatechuate decarboxylase activity
(b3) A DNA encoding a polypeptide comprising the amino acid sequence of SEQ ID NO: 4
(b4) A DNA encoding a polypeptide comprising an amino acid sequence that has 90% or more identity to SEQ ID NO: 4, the polypeptide having protocatechuate decarboxylase activity
(b5) A DNA encoding a polypeptide comprising an amino acid sequence that has 1 to 40 amino acids deleted, substituted, inserted, or added in the amino acid sequence of SEQ ID NO: 4, the polypeptide having protocatechuate decarboxylase activity

5. The *Hydrogenophilus* bacterium transformant according to claim 3 or 4, wherein the catechol 1,2-dioxygenase gene is any one of the following DNAs (c1) to (c5).
(c1) A DNA comprising the nucleotide sequence of SEQ ID NO: 5
(c2) A DNA comprising a nucleotide sequence that has 90% or more identity to SEQ ID NO: 5 and encoding a polypeptide having catechol 1,2-dioxygenase activity
(c3) A DNA encoding a polypeptide comprising the amino acid sequence of SEQ ID NO: 6
(c4) A DNA encoding a polypeptide comprising an amino acid sequence that has 90% or more identity to SEQ ID NO: 6, the polypeptide having catechol 1,2-dioxygenase activity
(c5) A DNA encoding a polypeptide comprising an amino acid sequence that has 1 to 30 amino acids deleted, substituted, inserted, or added in the amino acid sequence of SEQ ID NO: 6, the polypeptide having catechol 1,2-dioxygenase activity

6. The *Hydrogenophilus* bacterium transformant according to any one of claims 3 to 5, wherein the 3-dehydroshikimate dehydratase gene is any one of the following DNAs (d1) to (d5).
(d1) A DNA comprising the nucleotide sequence of SEQ ID NO: 7
(d2) A DNA comprising a nucleotide sequence that has 90% or more identity to SEQ ID NO: 7 and encoding a polypeptide having 3-dehydroshikimate dehydratase activity
(d3) A DNA encoding a polypeptide comprising the amino acid sequence of SEQ ID NO: 8
(d4) A DNA encoding a polypeptide comprising an amino acid sequence that has 90% or more identity to SEQ ID NO: 8, the polypeptide having 3-dehydroshikimate dehydratase activity
(d5) A DNA encoding a polypeptide comprising an amino acid sequence that has 1 to 60 amino acids deleted, substituted, inserted, or added in the amino acid sequence of SEQ ID NO: 8, the polypeptide having 3-dehydroshikimate dehydratase activity

7. The *Hydrogenophilus* bacterium transformant according to any one of claims 3 to 6, wherein the *Hydrogenophilus* bacterium is *Hydrogenophilus thermoluteolus*.

8. A method of producing muconic acid or catechol, comprising a step of culturing the *Hydrogenophilus* bacterium transformant according to any one of claims 3 to 7.

9. A positive selection marker for a *Hydrogenophilus* bacterium, comprising the following DNA (e1) or (e2).
(e1) A DNA comprising the nucleotide sequence of SEQ ID NO: 11
(e2) A DNA comprising a nucleotide sequence that has 90% or more identity to SEQ ID NO: 11 and encoding a polypeptide having activity of imparting bleomycin resistance to a *Hydrogenophilus* bacterium
